# EUROPEAN PATENT SPECIFICATION

(11) **EP 1 087 006 B1**
(45) Date of publication and mention of the grant of the patent: **15.10.2003**
(21) Application number: 00810848.2
(22) Date of filing: 19.09.2000
(51) Int. Cl.: C09K 11/06, H05B 33/14, C08K 5/3415, C07D 487/04, C09B 57/00

(54) **Electroluminescent devices comprising diketopyrrolopyrroles**
Elektrolumineszierende Anordungen mit Diketopyrrolopyrrolen
Dispositifs électroluminescents comprenant des dicétopyrrolopyrroles

(30) Priority: 27.09.1999 EP 99810868
(43) Date of publication of application: 28.03.2001
(60) Divisional application: 03009036.9
(73) Proprietor: Ciba Specialty Chemicals Holding Inc., 4057 Basel (CH)
(72) Inventor: Otani, Junji, Kobe-shi, Hyogo 657-0016 (JP); Yamamoto, Hiroshi, Takarazuka-shi, Hyogo 665-0864 (JP); Dan, Norihisa, Kyoto-shi, Kyoto 612-8492 (JP); Iqbal, Abul, 1732 Arconciel (CH); Moretti, Robert, 1213 Petit-Lancy (CH)

(56) References cited:
- EP-A- 0 133 156
- EP-A- 0 353 184
- EP-A- 0 499 011
- EP-A- 0 563 009
- EP-A- 0 811 625
- WO-A-96/08537
- WO-A-98/25927
- WO-A-98/32802
- PATENT ABSTRACTS OF JAPAN vol. 015, no. 072 (C-0808), 20 February 1991 (1991-02-20) & JP 02 296891 A (RICOH CO LTD), 7 December 1990 (1990-12-07)
- PATENT ABSTRACTS OF JAPAN vol. 018, no. 145 (C-1178), 10 March 1994 (1994-03-10) & JP 05 320633 A (SUMITOMO CHEM CO LTD), 3 December 1993 (1993-12-03)
- PATENT ABSTRACTS OF JAPAN vol. 1997, no. 05, 30 May 1997 (1997-05-30) & JP 09 003448 A (TOYO INK MFG CO LTD), 7 January 1997 (1997-01-07)

## Description

The present invention relates to an electroluminescent device comprising in this order
(a) an anode
(b) a hole transporting layer
(c) a light-emitting layer
(d) optionally an electron transporting layer and
(e) a cathode
and a light-emitting substance, wherein the light-emitting substance is a diketopyrrolopyrrole ("DPP") represented by formula I or formula III 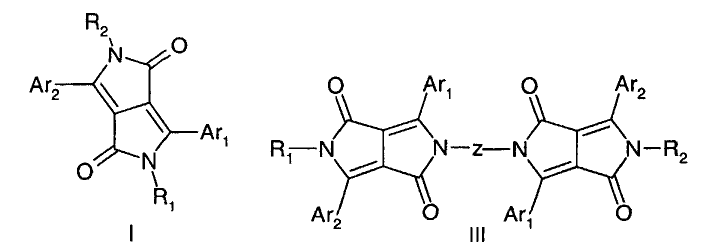 wherein R₁ and R₂, independently from each other, stand for C₁₋₂₅-alkyl, allyl which can be substituted one to three times with C₁-C₃alkyl or Ar₃, or -CR₃R₄-(CH₂)ₘ-Ar₃, wherein R₃ and R₄ substituted one to three times with C₁-C₃ alkyl,
Ar₃ stands for phenyl or 1- or 2-naphthyl which can be substituted one to three times with C₁-C₈alkyl, C₁-C₈alkoxy, halogen or phenyl, which can be substituted with C₁-C₈alkyl or C₁-C₈alkoxy one to three times, and m stands for 0, 1, 2, 3 or 4, Ar₁ and Ar₂, independently from each other, stand for 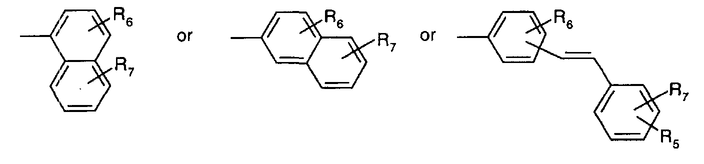 or 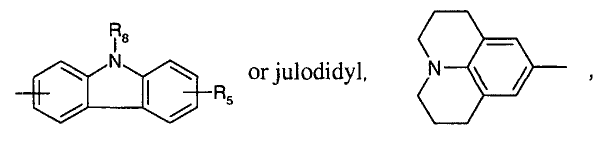 which can be substituted one to four times C₁-C₄alkyl, C₁-C₄alkoxy, or phenyl or 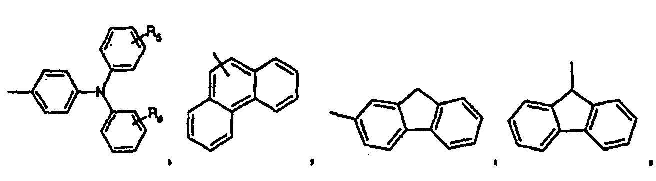 or 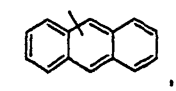 wherein R₅, R₆ and R₇, independently from each other, stand for hydrogen, cyano, halogen, C₁-C₆alkyl, -NR₈R₉, -OR₁₀, -S(O)ₙR₈, -Se(O)ₙR₈, or phenyl, which can be substituted one to three times with C₁-C₈alkyl or C₁-C₈alkoxy,
wherein R₈ and R₉, independently from each other, stand for hydrogen, phenyl, C₁-C₂₅-alkyl, C₅-C₁₂-cycloalkyl, -CR₃R₄-(CH₂)ₘ-Ph, R₁₀, wherein R₁₀ stands for C₈-C₂₄-aryl, or a saturated or unsaturated heterocyclic radical comprising five to seven ring atoms, wherein the ring consists of carbon atoms and one to three hetero atoms selected from the group consisting of nitrogen, oxygen and sulfur, wherein Ph, the aryl and heterocyclic radical can be substituted one to three times with C₁-C₈alkyl, C₁-C₈alkoxy, or halogen, or R₈ and R₉ stand for -C(O)R₁₁, wherein R₁₁ can be C₁-C₂₅-alkyl, C₅-C₁₂-cycloalkyl, R₁₀, -OR₁₂ or -NR₁₃R₁₄, wherein R₁₂, R₁₃, and R₁₄ stand for C₁-C₂₆-alkyl, C₅-C₁₂-cycloalkyl, C₆-C₂₄-aryl,
or
a saturated or unsaturated heterocyclic radical comprising five to seven ring
atoms, wherein the ring consists of carbon atoms and one to three hetero atoms selected from the group consisting of nitrogen, oxygen and sulfur, wherein the aryl and heterocyclic radical can be substituted one to three times with C₁-C₈alkyl or C₁-C₈alkoxy, or -NR₈R₉ stands for a five- or sixmembered heterocyclic radical in which R₈ and R₉ together stand for tetramethylene, pentamethylene, -CH₂-CH₂-O-CH₂-CH₂-, or -CH₂-CH₂-NR₅-CH₂-CH₂-, preferably -CH₂-CH₂-O-CH₂-CH₂-, and n stands for 0, 1, 2 or 3, and wherein Z stands for a diradical selected from the group consisting of a single bond, C₂-C₆alkylene, which can be substituted one to three times with C₁-C₄alkyl, C₁-C₄alkoxy, or phenyl, phenylene or naphthylene.

Thin film type electroluminescent devices usually consist essentially of a pair of electrodes and at least one charge transporting layer in between. Usually two charge transporting layers, a hole transporting layer (next to the anode) and an electron transporting layer (next to the cathode) are present. Either one of them contains - depending on its properties as hole-transporting or electron-transporting material - an inorganic or organic fluorescence substance as light-emitting material. It is also common, that a light-emitting material is used as an additional layer between the hole-transporting and the electron-transporting layer.

It is presently common to prepare organic electroluminescent ("EL") devices which contain an organic fluorescent substance by a vacuum evaporation process, e.g. described in Appl. Phys. Lett., 51, 913 (1987). In general, two types of such vacuum evaporation processes are applied according to the constitution of light emitting material: a one-component type process and a two-components type (or "Host-Guest type" or "binary system") process (e.g. described in J. Appl. Phys., 65, 3610 (1989)).

For emitting a light of red, green or blue color in a one-component system, the light emitting materials themselves have to emit an intense fluorescence of red, green or blue color. Further, a vacuum evaporation process has to give a deposited film of uniform quality, and the film thus formed has to be endowed with appropriate ("carrier") mobility for positive holes and/or electrons i.e. properties of a semiconductor.

Numerous materials emitting light in the green- or blue-colored region are known.

JP-B2 2,749,407 (Pioneer Electron Corp. & Nippon Kayaku Co. Ltd.) describes as a light emitting material N,N'-bis(2,5-di-tert.-butylphenyl)-3,4,9,10-perylenedicarboximide. However, its luminance is as low as 27 cd/m², which is insufficient for commercial applications.

JP-A2 2,296,891 (Ricoh) claims an electroluminescent element comprising a positive electrode, a negative electrode and one organic compound layer or a plurality of organic compound layers held between the positive and negative electrodes, but no hole transporting substance. At least one layer of said organic compound layers is a layer containing a pyrrolopyrrole compound represented by the following formula II 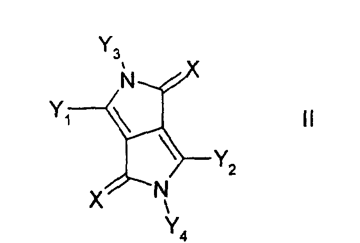 wherein Y₁ and Y₂ independently from each other represent a substituted or unsubstituted alkyl, cycloalkyl or aryl group, Y₃ and Y₄ independently represent a hydrogen atom or a substituted or unsubstituted alkyl or aryl group, and X represents an oxygen or a sulfur atom. Only four compounds are mentioned explicitly, namely wherein X stands for oxygen in all cases, and wherein (a) Y₃ = Y₄ = methyl and Y₁ = Y₂ = p-tolyl, (b) Y₃ = Y₄ = methyl and Y₁ = Y₂ = hydrogen, (c) Y₃ = Y₄ = hydrogen and Y₁ = Y₂ = p-tolyl, and (d) Y₃ = Y₄ = Y₁ = hydrogen and Y₂ = p-chlorophenyl. However, according to JP-A2 5,320,633 (see below), a follow-up study of the same inventors revealed that an emission of light is only observed, if the DPP-compounds II are used together with other compounds. This observation is supported by comparative example 2 of JP-A2 5,320,633, which shows that no emission is observed, if DPP II is used alone, i.e. without the addition of tris(8-hydroxyquinolinato)aluminium ("Alq₃").

JP-A2 5,320,633 (Sumitomo) claims an organic EL device having a light emitting layer comprising a light emitting material in an amount of 0.005 to 15 parts by weight of a DPP-compound between a pair of electrodes, wherein at least one electrode being transparent or semi-transparent. Although the main claim is silent about the use of Alq₃, it is clear from the specification and the examples, especially from comparative example 2, that Alq₃ is an essential feature in the claimed EL element or device.

JP-A2 9003448 (Toyo Ink) claims an organic EL element having between a pair of electrodes a luminous layer containing a DPP-compound as electron-transporting material or an organic compound thin film layer including a luminous layer and an electron-injecting layer wherein the electron-injecting layer contains a DPP compound as the electron-transporting material. In addition, another EL element further comprising a hole-injecting layer is claimed. The disadvantage of the claimed EL devices is that according to the examples always Alq₃ and a phenanthrene diamine (as hole-injecting material) have to be used.

EP-A-563 009 discloses an electroluminescent device comprising an anode, a hole-transporting-layer and a luminescent layer comprising 2,5-dimethyl-3,6-di(o-methoxyphenyl)pyrrolo[3,4-c]pyrrole and a cathode (see EP-A-563 009, Example 9).

EP-A 499,011 claims an organic EL element comprising a DPP compound, however, only systems having no electron-transporting layers are verified. Further, only highly crystalline organic pigments should be employed for a light emitting material. However, one of the requirements for light emitting materials is its morphological stability. Crystalline materials show a tendency to be morphologically modulated in the evaporated film. This becomes a disadvantage for ensuring device durability.

Usually in Host-Guest type light emitting materials, the sensitized fluorescence caused by the Förster type excitation energy transfer from Host to Guest is utilized. Accordingly, in addition to the abovementioned conditions, it is also important for this type of materials to fulfill the condition that the fluorescent spectrum of the Host in the solid state overlaps with the light absorption spectrum of the Guest in the solution state.

As to green-colored light emission, so high an EL emitting efficiency as >10 Im/W is achieved by using Alq₃ as Host and a quinacridone derivative as Guest. Such a system is used in practice in monocolor displays.

With regard to blue-colored light emission, Appl. Phys. Lett., 67, 3853 (1995) reports that a high EL emitting efficiency of 1.5 lm/W can be achieved by using a distyryl derivative as Host and an amino-substituted distyryl derivative as Guest. As above, the Guest material is not required to have a high carrier mobility, and therefore not required to be a semiconductor.

Many of the known orange-, red- or yellow-colored fluorescent dyes for use as dyestuff laser have a high fluorescence quantum yield in the solution state. Their Stokes shift, however, is generally small. That is, most of the yellow-, orange- or red-colored fluorescent dyes absorb yellow, orange or red light in the solution state and emit a yellow-, orange- or red-colored fluorescence. Accordingly, if a yellow-, orange- or red-colored fluorescent dye is considered as Guest, the Host material should be a material emitting a yellow-, orange- or redcolored fluorescence in the solid state, as designated by the conditions necessary forrealization of a sensitized fluorescence through Förster type excitation energy transfer.

Further, compatibility with the Guest must also be taken into account in addition to the conditions similar to those required of one-component type light emitting materials. In conclusion, the Host material must be a yellow-, orange- to red-colored solid fluorescent material in order to realize a yellow-, orange- or red-colored organic EL luminescence in a two-components system. At the present stage, however, no satisfactory yellow-, orange- or red-light emitting material is known for the same reason as in the one-component system.

EP-A-0 811 625 relates to a process for preparing diketopyrrolopyrrole derivatives. In Example 8 of EP-A-0 811 625 the preparation of 2,5-di(hydroxymethyl)-3,6-di(biphen-1-yl)pyrrolo[3,4-c]pyrrole-1,4-dione is disclosed.

WO98/25927 discloses liquid crystalline N-substituted 1,4-diketo-3,6-diaryl-pyrrolo-[3,4-c]pyrroles, 1,4-diketo-3,6-dialkylpyrrolo[3,4-c]pyrroles and 1,4-diketo-3-alkyl-6-arylpyrrolo-[3,4-c]pyrroles.

EP-A 648770 describes solid fluorescent, soluble latent pigments. However, latent pigments cannot be used for the abovementioned vacuum evaporation processes because they are transformed into insoluble non-fluorescent DPP pigments upon heating.

Hence, the object of this invention was to provide electroluminescent devices emitting yellow, orange or red light, wherein organic light-emitting materials should be used fulfilling
- intense photoluminescence in the solid state for the one-component system, and/or the Host in the binary system, and in the solution state for the Guest in the binary system,
- carrier mobility for a positive hole and/or an electron,
- necessary properties for vacuum evaporation and deposition (such as ability to sublime or evaporate),
- the ability for a homogeneous film formation,
- the property of exhibiting a "pure" color,
- the ability that the electronic potential should match with the electrodes and/or with the substances adjacent to,
- compatibility of the solid host and the molecular guest in case binary systems are desired,
- high durability (thermal, electrical etc.) and morphological stability.

Accordingly, the abovementioned electroluminescent devices were found. In addition, a process for its preparation, and new light-emitting materials were found, too.

Typical constitutions of latest organic electroluminescent devices are:
(i) an anode/a hole transporting layer/an electron transporting layer/a cathode, in which compounds I are used either as positive-hole transport compounds, which is exploited to form the light emitting and hole transporting layers , or as electron transport compounds, which can be exploited to form the light-emitting and electron transporting layers, and
(ii) an anode/a hole transporting layer/a light-emitting layer/an electron transporting layer/a cathode,
   in which the compounds I form the light-emitting layer regardless of whether they exhibit positive-hole or electron transport properties in this constitution.

It is possible that the light-emitting layer can consist of two or more fluorescent substances of formula I for energy donor(s) and/or energy acceptor(s).

The devices can be prepared in several ways. Usually, vacuum evaporation is extensively used for the preparation. Preferably, the organic layers are laminated in the above order on a commercially available indium-tin-oxide ("ITO") glass substrate held at room temperature, which works as the anode in the constitutions. The membrane thickness is preferably in the range of 1 to 10,000 nm, more preferably 1 to 5,000 nm, more preferably 1 to 1,000 nm, more preferably 1 to 500 nm. The cathode metal such as Mg/Ag alloy and Li-Al binary system of ca. 200 nm is laminated on the top of the organic layers. The vacuum during the deposition is preferably less than 0.1333 Pa (1x 10⁻³ Torr), more preferably less than 1.333x 10⁻³ Pa (1x 10⁻⁵ Torr), more preferably less than 1.333x 10⁻⁴ Pa (1x 10⁻⁶ Torr).

As anode usual anode materials which possess high work function such as metals like gold, silver, copper, aluminum, indium, iron, zinc, tin, chromium, titanium, vanadium, cobalt, nickel, lead, manganese, tungsten and the like, metallic alloys such as magnesium/copper, magnesium/silver, magnesium/aluminum, aluminum/indium and the like, semiconductors such as Si, Ge, GaAs and the like, metallic oxides such as indium-tin-oxide ("ITO"), ZnO and the like, metallic compounds such as Cul and the like, and furthermore, electroconducting polymers such polyacetylene, polyaniline, polythiophene, polypyrrole, polyparaphenylene and the like, preferably ITO, most preferably ITO on glass as substrate can be used.
Of these electrode materials, metals, metallic alloys, metallic oxides and metallic compounds can be transformed into electrodes, for example, by means of the sputtering method. In the case of using a metal or a metallic alloy as a material for an electrode, the electrode can be formed also by the vacuum deposition method. In the case of using a metal or a metallic alloy as a material forming an electrode, the electrode can be formed, furthermore, by the chemical plating method (see for example, Handbook of Electrochemistry, pp 383-387, Mazuren, 1985). In the case of using an electroconducting polymer, an electrode can be made by forming it into a film by means of anodic oxidation polymerization method onto a substrate which is previously provided with an electroconducting coating. The thickness of an electrode to be formed on a substrate is not limited to a particular value, but, when the substrate is used as a light emitting plane, the thickness of the electrode is preferably within the range of from 1 nm to 100 nm, more preferably, within the range of from 5 to 50 nm so as to ensure transparency.

In a preferred embodiment ITO is used on a substrate having an ITO film thickness in the range of from 10 nm (100 Å) to 1 µm (10000 Å), preferably from 20 nm (200 Å) to 500 nm (5000 Å). Generally, the sheet resistance of the ITO film is chosen in the range of not more than 100 Ω/cm², preferred from not more than 50 Ω/cm².

Such anodes are commercially available from e.g. Japanese manufacturers such as Geomatech Co.Ltd., Sanyo Vacuum Co. Ltd., Nippon Sheet Glass Co. Ltd.

As substrate either an electronconducting or electrically insulating material can be used. In case of using an electroconducting substrate, a light emitting layer or a positive hole transporting layer is directly formed thereupon, while in case of using an electrically insulating substrate, an electrode is firstly formed thereupon and then a light emitting layer or a positive hole transporting layer is superposed.

The substrate may be either transparent, semi-transparent or opaque. However, in case of using a substrate as an indicating plane, the substrate must be transparent or semi-transparent.

Transparent electrically insulating substrates are, for example, inorganic compounds such as glass, quartz and the like, organic polymeric compounds such as polyethylene, polypropylene, polymethylmethacrylate, polyacrylonitrile, polyester, polycarbonate, polyvinylchloride, polyvinylalcohol, polyvinylacetate and the like. Each of these substrates can be transformed into a transparent electroconducting substrate by providing it with an electrode according to one of the methods described above.

As examples of semi-transparent electrically insulating substrates, there are inorganic compounds such as alumina, YSZ (yttrium stabilized zirconia) and the like, organic polymeric compounds such as polyethylene, polypropylene, polystyrene, epoxy resin and the like. Each of these substrates can be transformed into a semi-transparent electroconducting substrate by providing it with an electrode according to one of the abovementioned methods.

As examples of opaque electroconducting substrates, there are metals such as aluminum, indium, iron, nickel, zinc, tin, chromium, titanium, copper, silver, gold, platinum and the like, various elctroplated metals, metallic alloys such as bronze, stainless steel and the like, semiconductors such as Si, Ge, GaAs, and the like, electroconducting polymers such as polyaniline, polythiophene, polypyrrole, polyacetylene, polyparaphenylene and the like.

A substrate can be obtained by forming one of the above listed substrate materials to a desired dimension. It is preferred that the substrate has a smooth surface. Even if it has a rough surface, however, it will not cause any problem for practical use, provided that it has round unevenness having a curvature of not less than 20 µm. As for the thickness of the substrate, there is no restriction as far as it ensures sufficient mechanical strength. As cathode usual cathode materials which possess low work function such as alkali metals, earth alkaline metals, group 13 elements, silver, and copper as well as alloys or mixtures thereof such as sodium, lithium, potassium, sodium-potassium alloy, magnesium, magnesium-silver alloy, magnesium-copper alloy, magnesium-aluminum alloy, magnesium-indium alloy, aluminum, aluminum-aluminum oxide alloy, aluminum-lithium alloy, indium, calcium, and materials exemplified in EP-A 499,011 such as electroconducting polymers e.g. polypyrrole, polythiophene, polyaniline, polyacetylene etc., preferably Mg/Ag alloys, or Li-Al compositions can be used.

In a preferred embodiment magnesium-silver alloy or a mixture of magnesium and silver mixture, or lithium-aluminum alloy or a mixture of lithium and aluminum can be used in a film thickness in the range of from 10 nm (100 Å) to 1 µm (10000 Å), preferably from 20 nm (200 Å) to 500 nm (5000 Å).

Such cathodes can be deposited on the foregoing electron transporting layer by known vacuum deposition techniques described above.

In a preferred ambodiment of this invention a light-emitting layer can be used between the hole transporting layer and the electron transporting layer. Usually it is prepared by forming a thin film of a DPP compound of formula I on the hole transporting layer.

As methods for forming said thin film, there are, for example, the vacuum deposition method, the spin-coating method, the casting method, th Langmuir-Blodgett ("LB") method and the like. Among these methods, the vacuum deposition method, the spin-coating method and the casting method are particularly preferred in view of ease in operation and cost.

In case of forming a thin film using a DPP compound I by means of the vacuum deposition method, the conditions under which the vacuum deposition is carried out are usually strongly dependent on the properties, shape and crystalline state of the compound. However, optimum conditions can be selected for example within the range of from 100 to 400°C in temperature for the heating boat, -100 to 350°C in substrate temperature, 1.33x10⁴ Pa (1x10² Torr) to 1.33x10⁻⁴ Pa (1x10⁻⁶ Torr) in pressure and 1 pm to 6 nm/sec in deposition rate.

In an organic EL element, the thickness of the light emitting layer thereof is one of the factors determining its light emission properties. For example, if a light emitting layer is not sufficiently thick, a short circuit can occur quite easily between two electrodes sandwiching said light emitting layer, and therefor, no EL emission is obtained. On the other hand, if the light emitting layer is excessively thick, a large potential drop occurs inside the light emitting layer because of its high electrical resistance, so that the threshold voltage for EL emission increases. Accordingly, it is necessary to limit the thickness of an organic light emitting layer within the range of from 5 nm to 5 µm. A preferable thickness is within the range of from 10 nm to 500 nm.

In the case of forming a light emitting layer by using the spin-coating method and the casting method, the coating can be carried out using a solution prepared by dissolving the DPP I in a concentration of from 0.0001 to 90% by weight in an appropriate organic solvent such as benzene, toluene, xylene, tetrahydrofurane, methyltetrahydrofurane, N,N-dimethylformamide, dichloromethane, dimethylsulfoxide and the like. Herein, the higher the concentration of DPP I, the thicker the resulting film, while the lower the concentration, the thinner the resulting film. However, if the concentration exceeds 90% by weight, the solution usually is so viscous that it no longer permits forming a smooth and homogenous film. On the other hand, as a rule, if the concentration is less than 0.0001% by weight, the efficiency of forming a film is too low to be economical. Accordingly, a preferred concentration of DPP I is within the range of from 0.01 to 80% by weight.

In the case of using the above spin-coating or casting method, it is possible to further improve the homogeneity and mechanical strength of the resulting layer by adding a polymer binder in the solution for forming the light emitting layer. In principle, any polymer binder may be used, provided that it is soluble in a solvent in which DPP I is dissolved. Examples of such polymer binders are polycarbonate, polyvinylalcohol, polymethacrylate, polymethylmethacrylate, polyester, polyvinylacetate, epoxy resin and the like. A solution for forming a light emitting layer may have any concentrations of DPP I, of a polymer binder and solvent. However, if the solid content composed of the polymer binder and DPP I exceeds 99% by weight, the fluidity of the solution is usually so low that it is impossible to form a light emitting layer excellent in homogeneity. On the other hand, if the content of DPP I is substantially smaller than that of the polymer binder, in general the electrical resistance of said layer is very large, so that it does not emit light unless a high voltage is applied thereto. Furthermore, since the concentration of DPP I in the layer is small in this case, its light emission efficiency is relatively low. Accordingly, the preferred composition ratio of a polymer binder to DPP I is chosen within the range of from 10:1 to 1:50 by weight, and the solid content composed of both components in the solution is preferably within the range of from 0.01 to 80% by weight, and more preferably, within the range of about 0.1 to 60% by weight.

In the case of forming a light emitting layer by the spin-coating method or casting method, the thickness of said layer may be selected in the same manner as in the case of forming a light emitting layer by the vacuum deposition method. That is, the thickness of the layer preferably is chosen within the range of from 5 nm to 5 µm, and more preferably, within the range of from 10 nm to 500 nm.

As hole-transporting layers known organic hole transporting compounds such as polyvinyl carbazole, 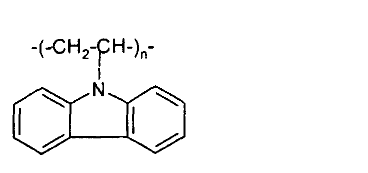 a TPD compound disclosed in J.Amer.Chem.Soc. 90 (1968) 3925 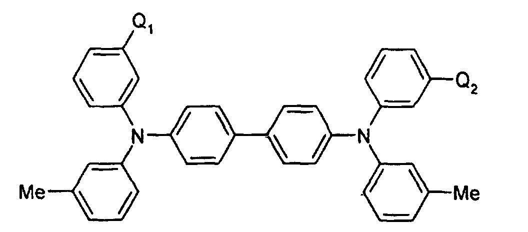 wherein Q₁ and Q₂ each represent a hydrogen atom or a methyl group;
a compound disclosed in J. Appl. Phys. 65(9) (1989) 3610 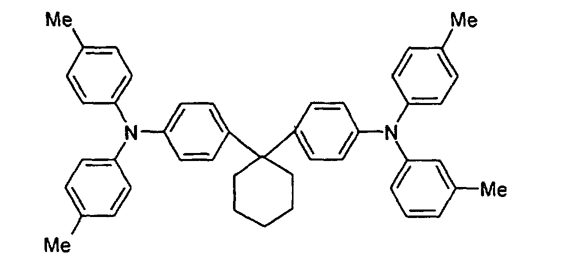 a stilbene based compound 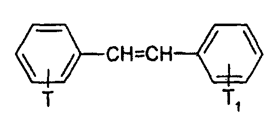 wherein T and T₁ stand for an organic rest
a hydrazone based compound 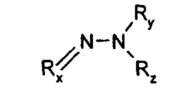 and the like.

Compounds to be used as a positive hole transporting material are not restricted to the above listed compounds. Any compound having a property of transporting positive holes can be used as a positive hole transporting material such as triazole derivatives, oxadiazole. derivatives, imidazole derivatives, polyarylalkane derivatives, pyrazoline derivative, pyrazolone derivatives, phenylene diamine derivatives, arylamine derivatives, amino substituted chalcone derivatives, oxazole derivatives, stilbenylanthracene derivatives, fluorenone derivatives, hydrazone derivatives, stilbene derivatives, copolymers of aniline derivatives, electro-conductive oligomers, particularly thiophene oligomers, porphyrin compounds, aromatic tertiary amine compounds, stilbenyl amine compounds etc. Particularly, aromatic tertiary amine compounds such as N,N,N',N'-tetraphenyl-4,4'-diaminobiphenyl, N,N'-diphenyl-N,N'-bis(3-methylphenyl)- 4,4'-diaminobiphenyl (TPD), 2,2'-bis(di-p-torylaminophenyl)propane, 1,1'-bis(4-di-torylaminophenyl)-4-phenylcyclohexane, bis(4-dimethylamino-2-methylphenyl)phenylmethane, bis(4-di-p-tolylaminophenyl)phenylmethane, N,N'-diphenyl-N,N'-di(4-methoxyphenyl)-4,4'-diaminobiphenyl, N,N,N',N'-tetraphenyl-4,4'-diaminodiphenylether, 4,4'-bis(diphenylamino)quaterphenyl, N,N,N-tri(p-tolyl)amine, 4-(di-p-tolylamino)-4'-[4-(di-p-tolylamino)stilyl]stilbene, 4-N,N-diphenylamino-(2-diphenylvinyl)benzene, 3-methoxy-4'-N,N-diphenylaminostilbene, N-phenylcarbazole etc.

Furthermore, 4,4'-bis[N-(1-naphtyl)-N-phenylamino]biphenyl disclosed in US 5,061,569, the compounds in which three triphenylamine units are bound to a nitrogen atom like "star-burst" structure e.g. 4,4',4"-tris[N-(3-methylphenyl)-N-phenylamino]triphenylamine disclosed in EP-A 508,562.

A positive hole transporting layer can be formed by preparing an organic film containing at least one positive hole transporting material on the anode. The positive hole transporting layer can be formed by the vacuum deposition method, the spin-coating method, the casting method, the LB method and the like. Of these methods, the vacuum deposition method, the spin-coating method and the casting method are particularly preferred in view of ease and cost.

In the case of using the vacuum deposition method, the conditions for deposition may be chosen in the same manner as described for the formation of a light emitting layer (see above). If it is desired to form a positive hole transporting layer comprising more than one positive hole transporting material, the coevaporation method can be employed using the desired compounds.

In the case of forming a positive hole transporting layer by the spin-coating method or the casting method, the layer can be formed under the conditions described for the formation of the light emitting layer (see above).

As in the case of forming a light emitting layer using a solution containing a polymer binder, a smoother and more homogeneous positive hole transporting layer can be formed by using a solution containing a binder and at least one positive hole transporting material. The coating using such a solution can be performed in the same manner as in cases of forming a light emitting layer using a polymer binder. Any polymer binder may be used, provided that it is soluble in a solvent in which at least one positive hole transporting material is dissolved. Examples of appropriate polymer binders and of appropriate and preferred concentrations are given above when describing the formation of a light emitting layer.

The thickness of a positive hole transporting layer is preferably chosen in the range of from 0.5 to 1000 nm, preferably from 1 to 100 nm, more preferably from 2 to 50 nm.

As electron transporting materials for an electron-transporting layer it is preferred to have a high electron injection efficiency from the cathode and a high electron mobility. The following materials can be exemplified for electron transporting materials: tris(8-hydroxyquinolinato)-aluminum(III) and its derivatives, bis(10-hydroxybenzo[h]quinolinolato)beryllium(II) and its derivatives, oxadiazole derivatives such as 2-(4-biphenyl)-5-(4-tert.-butylphenyl)-1,3,4-oxadiazole and its dimer systems such as 1,3-bis(4-tert.-butylphenyl-1,3,4)oxadiazolyl)biphenylene and 1,3-bis(4-tert.-butylphenyl-1,3,4-oxadiazolyl)phenylene, triazole derivatives, phenanthroline derivatives or perylene tetracarboxylic acid derivatives such as disclosed in Appl. Phys. Lett. 48 (2) (1986) 183.

An electron transporting layer can be formed by preparing an organic film containing at least one electron transporting material on the hole transporting layer or on the light-emitting layer. The electron transporting layer can be formed by the vacuum deposition method, the spin-coating method, the casting method, the LB method and the like.

As in the case of forming a light emitting layer or a positive hole transporting layer by using a solution containing a polymer binder, a smoother and more homogeneous electron transporting layer can be formed by using a solution containing a binder and at least one electron transporting material.

The thickness of an electron transporting layer is preferably chosen in the range of from 0.5 to 1000 nm, preferably from 1 to 100 nm, more preferably from 2 to 50 nm.

Yellow to red fluorescent light-emitting compounds means that the used light-emitting compounds preferably have a fluorescence emission maximum in the range of from 500 to 780, more preferably from 520 to 750, more preferred from 540 to 700 nm. Further, the inventive compounds preferably exhibit an absorption maximum in the range of 450 to 580 nm.

The light-emitting compounds I usually exhibit a fluorescence quantum yield ("FQY") in the range of from 1 > FQY ≥ 0.3 (measured in aerated toluene or DMF). Further, in general, the inventive compounds I exhibit a molar absorption coefficient in the range of from 5000 to 100000.

A preferred embodiment relates to DPP-compounds I, wherein R₁ = R₂, and Ar₁ = Ar₂, particularly preferred wherein in addition to the above R₃ = R₄ = H, m = 0 and n = 0, most preferred are DPP-compounds in which
(a) R₁ = R₂ = C₁-C₈alkyl, Ar₁ = Ar₂ = stilbene, R₇ = -NR₈R₉ in 4-position, R₅ = R₆ = hydrogen, and R₈ = R₉ = C₁-C₈alkyl or phenyl, or
(b) R₁ = R₂ = C₁-C₈alkyl, -(CH₂)ₘ-Ph, Ar₁ = Ar₂ = stilbene, R₅ = R₆ = hydrogen, R₇ = -SR₇, -OR₁₀, -N(R₈)₂ or unsubstituted or substituted phenyl in para-position, and R₈ = C₁-C₈alkyl, phenyl or a heterocyclic radical, both unsubstituted or substituted, or C₅-C₁₂-cycloalkyl, or
(c) R₁ = R₂ = -CH₂-Ph, wherein phenyl can be substituted with phenyl, naphthyl or C₁-C₄alkyl up to two times, Ar₁ = Ar₂ = 1- or 2-naphthyl, R₅ = R₆ = hydrogen, R₇ = hydrogen or -OMe, in case where Ar₁ = Ar₂ = 1- or 2-naphthyl, or, in all other cases, C₁-C₈alkyl or phenyl.

Particularly preferred DPP-compounds I are the following compounds: 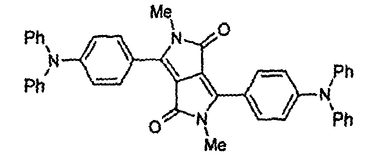 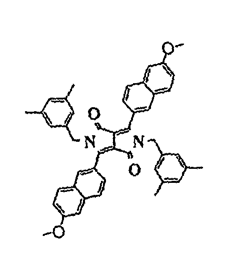 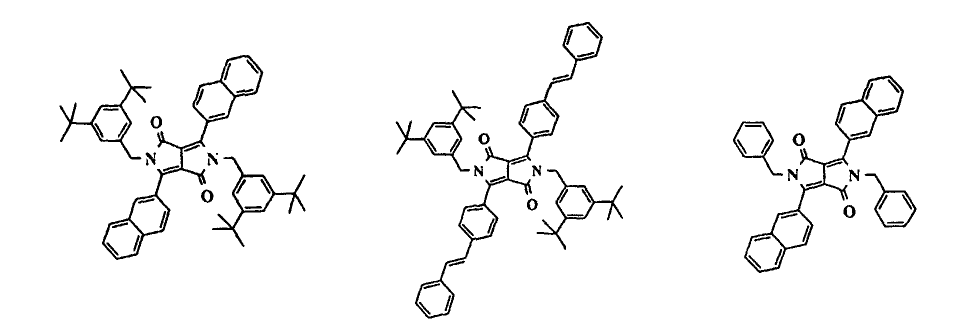 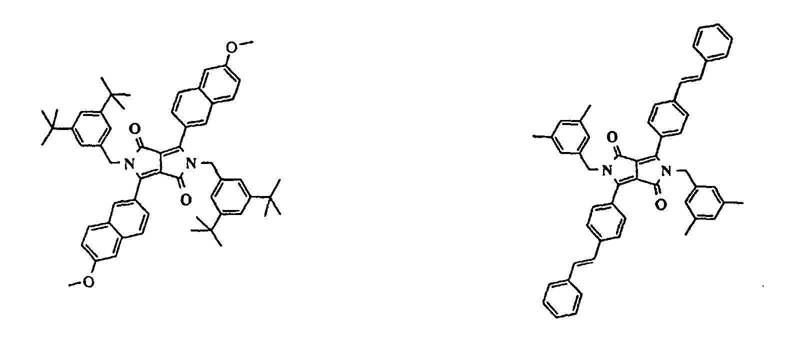 C₁-C₂₅alkyl is typically linear or branched - where possible - methyl, ethyl, n-propyl, isopropyl, n-butyl, sec.-butyl, isobutyl, tert.-butyl, n-pentyl, 2-pentyl, 3-pentyl, 2,2-dimethylpropyl, n-hexyl, n-heptyl, n-octyl, 1,1,3,3-tetramethylbutyl and 2-ethylhexyl, n-nonyl, decyl, undecyl, dodecyl, tetradecyl, pentadecyl, hexadecyl, heptadecyl, octadecyl, eicosyl, heneicosyl, docosyl, tetracosyl or pentacosyl, preferably C₁-C₈alkyl such as methyl, ethyl, n-propyl, isopropyl, n-butyl, sec.-butyl, isobutyl, tert.-butyl, n-pentyl, 2-pentyl, 3-pentyl, 2,2-dimethylpropyl, n-hexyl, n-heptyl, n-octyl, 1,1,3,3-tetramethylbutyl and 2-ethylhexyl, more preferably C₁-C₄alkyl such as typically methyl, ethyl, n-propyl, isopropyl, n-butyl, sec.-butyl, isobutyl, tert.-butyl; C₁-C₆alkyl stands for methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl; C₁-C₃alkyl stands for methyl, ethyl, n-propyl, or isopropyl.

C₁-C₈alkoxy is typically methoxy, ethoxy, n-propoxy, isopropoxy, n-butoxy, sec.-butoxy, isobutoxy, tert.-butoxy, n-pentoxy, 2-pentoxy, 3-pentoxy, 2,2-dimethylpropoxy, n-hexoxy, n-heptoxy, n-octoxy, 1,1,3,3-tetramethylbutoxy and 2-ethylhexoxy, preferably C₁-C₄alkoxy such as typically methoxy, ethoxy, n-propoxy, isopropoxy, n-butoxy, sec.-butoxy, isobutoxy, tert.-butoxy.

C₆-C₂₄aryl is typically phenyl, 1-naphthyl, 2-naphthyl, 4-biphenyl, phenanthryl, 2- or 9-fluorenyl or anthracenyl, preferably C₆-C₁₂aryl such as phenyl, 1-naphthyl, 2-naphthyl, 4-biphenyl.

C₇-C₂₄aralkyl is typically benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred C₇-C₁₂aralkyl such as benzyl, 2-benzyl-2-propyl, β-phenyl-ethyl, a,a-dimethylbenzyl, ω-phenyl-butyl, or ω,ω-dimethyl-ω-phenyl-butyl.

C₅-C₁₂cycloalkyl is typically cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, cyclooctyl.

Heteroaryl with five to seven ring atoms, wherein nitrogen, oxygen or sulfur are the possible hetero atoms, is typically an unsaturated heterocyclic radical with five to 18 atoms having at least six conjugated π-electrons such as thienyl, benzo[b]thienyl, dibenzo[b,d]thienyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, perimidinyl, phenanthrolinyl, phenazinyl, isothiazolyl, phenothiazinyl, isoxazolyl, furazanyl or phenoxazinyl, preferably the abovementioned mono- or bicyclic heterocyclic radicals.

The inventive DPP-compounds I can be synthesized according to methods well known in the art such as described in EP-A 133,156, e.g. in analogy to example 15.

A preferred embodiment of this invention relates to a process for the preparation of the inventive compounds I or III by treating in a first step the DPP derivative of formula Va or Vb 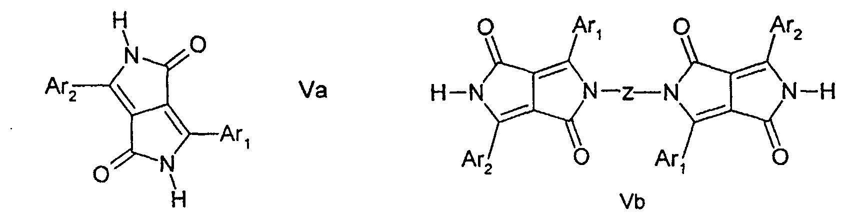 with a base, then, in a second step, treating the reaction mixture obtained in the first step with a alkylating agent, wherein in the first step the base is a hydride, an alkali metal alkoxide or a carbonate, and the alkylating agent is a sulfonate, tosylate, mesylate, carbonate, sulfate, or halogen compound of the formula (R₁)_{1 or 2}X, wherein X stands for SO₃-, (p-Me-phenyl)SO₂-, (2,4,6-trimethyl-phenyl)SO₂-, -CO₃-, -SO₄-, or halogen such as chlorine, bromine or iodine, preferably chlorine, bromine or iodine, particularly preferred for bromine or iodine, or a mixture of (R₁)_{1 or 2}X and (R₂)_{1 or 2}X.

As a hydride usually an alkali metal hydride such as sodium hydride, lithium hydride, or potassium hydride, as an alkali metal alkoxide in general an alkali metal C₁-C₄alkoxide such as sodium or potassium tert. butoxide, sodium tert.-amylate, and as a carbonate usually sodium or potassium carbonate can be used, preferably sodium hydride.

Usually, the first step of the preferred preparation of compound I or III starting from compound Va, resp. Vb, is carried out at a temperature in the range of from -25 to 100, preferably from 0 to 25°C.

Preferably, the reaction is carried out in the presence of a solvent, preferably a dipolar aprotic solvent such as carboxamides, lactams, urea derivatives, sulfones and nitrobenzene such as dimethyl formamide ("DMF"), dimethyl acetamide ("DMA"), N-methylpyrrolidone ("NMP"), N,N'-dimethylethylene urea and N,N'-dimethylpropylene urea.

In case a solvent is used, a weight ratio of solvent to DPP-compound is chosen in the range of from 100:1 1 to 5:1, preferably from 25:1 to 10:1.

In addition, it is preferred to carry out the first step in the presence of a phase transfer catalyst such as a tetra alkyl ammonium halide such as tetraethyl ammonium bromide.

Usually, a molar ratio of base to DPP-compound Va, resp. Vb, is chosen in the range of from 10:1 to 2:1, preferably from 4:1 to 2:1.

Preferably, a molar ratio of DPP-compound Va, resp.Vb, to the phase transfer catalyst is chosen in the range of from 100:1 1 to 5:1, preferably from 25:1 to 10:1.

Generally, the reaction time depends inter alia on the reactivity of the chosen reactants and the chosen temperature. As an example, if room temperature is chosen as reaction temperature, a reaction time is as a rule in the range of from 0.5 to 24 hours.

Preferably, the halogen compound R₁-X (or the aforementioned mixture) is added to the reaction mixture obtained in the first step in the same solvent used in the first step.
The reaction temperature in the second process step usually is chosen in the range of from 0 to 160, preferably from 25 to 110°C, depending on *inter alia* the desired reaction pressure and solvent used. The reaction time generally is chosen in the range of from 0.5 to 120, preferably from 12 to 60 hours.

As a rule the molar ratio of R,-X to DPP compound Va, resp.Vb, is chosen in the range of from 10:1 to 2:1, preferably from 4:1 to 2:1.

In case a solvent is used, the amount of solvent usually is chosen in the range of from 100:1 to 5:1, preferably from 25:1 to 10:1, based on the amount of halogen compound R₁-X. Further, preferably the same solvent is used as in the first step, if a solvent is used in the first step. If no solvent is used in the first step, the same solvents can be used as mentioned above.

The obtained reaction mixture can be worked up by applying methods well known in the art, e.g. by precipitating the product in the presence of an appropriate solvent such as water, and, if deemed necessary, by re-crystallization in an appropriate solvent such as ethanol. Other methods for example are the addition of an alcohol to quench the excess base followed by filtration.

Compounds Va are described e.g. in US 4,579,949, and/or can be prepared according to the method described therein, in which an appropriate nitrile is reacted with a corresponding dialkyl or diaryl succinate, e.g. NC-Ar₁ is reacted with sodium tert.-amyl alcohol followed by the addition of diisopropyl succinate. This method is preferred in case Ar, and/or Ar₂ stand for a biphenyl radical (i.e. R₅ and/or R₆ stand for phenyl or substituted phenyl in 4-position), or for the compounds described below (DPP VIa).

Compounds Vb can be prepared e.g. via the following route 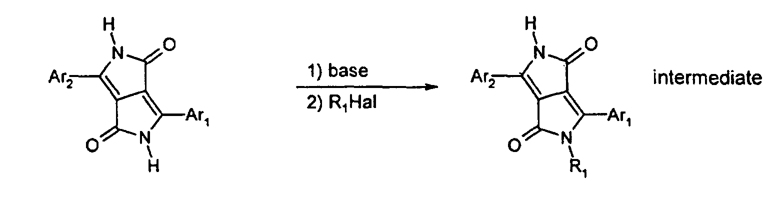 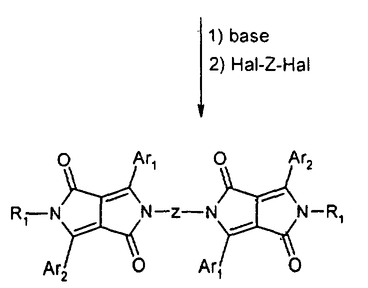 Of course, instead of using R₁Hal, a mixture of R₁-Hal and R₂-Hal can be used which then would lead to the general formula Vb. Usually, R₁Hal, resp. the mixture of R₁Hal and R₂Hal, is used in a molar amount in the range of from 0.4-0.6:1, related to the starting DPP derivative. Accordingly, generally the molar amount of Hal-Z-Hal/intermediate is chosen in the range of from 0.4-0.6:1.

Compounds I, resp. III, are also available in analogy to the method described in EP-A 353,184, which comprises reacting a DPP-compound of formula VIa or VIb 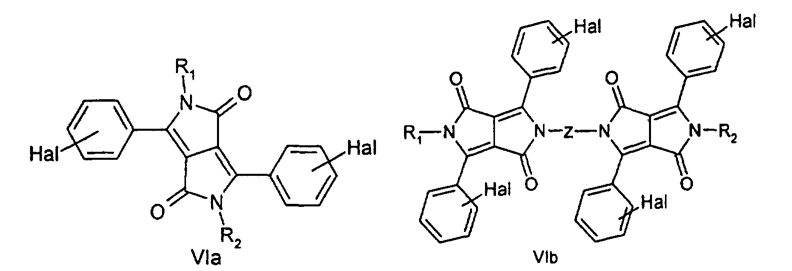 wherein Hal stands for halogen such as fluorine, chlorine, bromine or iodine, preferably chlorine or bromine, with a nucleophilic agent such as a secondary amine, HNR₈R₉, a thiol, HSR₈, or HS(O)ₙR₈, an alcohol, HOR₁₀, a diselenide, R₈(O)ₙSe-Se(O)ₙR₈, preferably in a molar ratio of DPP VIa, resp. VIb,:nucleophilic agent in the range of 1.2:1 to 0.8:1, or, if R₂ has the same meaning as R₁ in the range of from 1:2.5 to 1:1, in the presence of an anhydrous dipolar aprotic solvent, and of an anhydrous base in an amount in the range of from usually 0.1 to 15 moles per mole of the nucleophilic agent, at a temperature in the range of from usually 100 to 220°C and under a pressure generally in the range of from 100 to 300 kPa.

Examples of suitable anhydrous dipolar aprotic solvents are carboxamides, lactams, urea derivatives, sulfones and nitrobenzene such as DMF, DMA, NMP, N,N'-dimethylethylene urea and N,N'-dimethylpropylene urea.

Suitable anhydrous bases are e.g. anhydrous organic bases such as quinoline, or preferably, an excess of the secondary amine used for the amination, the aforementioned carbonates such as sodium or potassium carbonate and alkali metal hydrides such as sodium hydride. In case a diselenide, R₇(O)ₙSe-Se(O)ₙR₇, is used, an alkali metal hydride, preferably sodium hydride, has to be used as a base.

The corresponding 1- and 2-naphthyl-derivatives can be prepared analogously.

DPP-compounds VIa and VIb are known and/or can be prepared e.g. according to the method described in US 4,579,949, which methods comprises reacting a dialkyl or diaryl succinate with a nitrile, e.g. dimethyl succinate can be reacted with p-chloro benzonitrile according to example 6 in US 4,579,949 to yield the corresponding DPP compound VIa, in which Hal stands for chlorine.

Compounds R₁-X are commercially available or can be prepared by methods well known in the art.

A further embodiment of the invention on hand concerns a process for the preparation of the inventive compounds I resp. III
(a) in treating in a first step the DPP derivative of formula VIa, resp. VIb, with a nucleophilic agent such as a secondary amine, HNR₈R₉, a thiol, HSR₈, or HS(O)ₙR₈, an alcohol, HOR₁₀, a diselenide, R₈(O)ₙSe-Se(O)ₙR₈, preferably in a molar ratio of DPP VIa, resp. VIb,:nucleophilic agent in the range of 1.2:1 to 0.8:1, or, if R₂ has the same meaning as R, in the range of from 1:2.5 to 1:1, in the presence of an anhydrous dipolar aprotic solvent, and of an anhydrous base in an amount in the range of from usually 0.1 to 15 moles per mole of the nucleophilic agent, at a temperature in the range of from usually 100 to 220°C and under a pressure generally in the range of from 100 to 300 kPa, and optionally isolating the obtained compound V,
(b) then treating the obtained compound Va, resp. Vb, with a base, thereafter in a second step, treating the reaction mixture obtained in the first step of (b) with an usual alkylating agent, wherein in the first step of (b) the base is a hydride, an alkali metal alkoxide or a carbonate, and the alkylating agent is a sulfonate, tosylate, mesylate, carbonate, sulfate, or halogen compound of the formula (R₁)_{1 or 2}X, wherein X stands for SO₃-, (p-Me-phenyl)SO₂-, (2,4,6-trimethyl-phenyl)SO₂-, -CO₃-, -SO₄-, or halogen, or a mixture of (R₁)_{1 or 2}X and (R₂)_{1 or 2}X (it is evident, that the number of R₁-units (either one or two) in (R₁)_{1 or 2}X depends on the nature of the chosen rest X, i.e. there can be only two R₁-units if X stands for a divalent anion such as -CO₃-, -SO₄- etc.).

Water-soluble compounds I, resp.III, i.e. inventive compounds I or III being substituted with a functional group capable of increasing the solubility in water such as a tertiary amino group, SO₃⁻, or PO₄²⁻, can be prepared by using well-known methods in the art. The following routes are representative examples, and, hence, do not restrict the invention just to these examples: 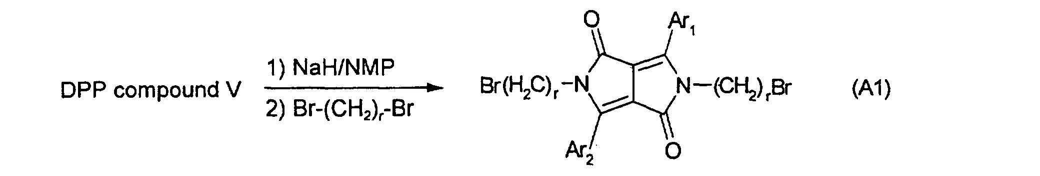 wherein r stands for an integer from usually 2 to 25; instead of linear alkyl groups, one could also use branched alkyl groups or aralkyl groups such as Br-(CH₂)ᵣ₁-aryl-(CH₂)ᵣ₂-Br, r₁ and r₂ usually being whole numbers in the range of from 0 to 10; 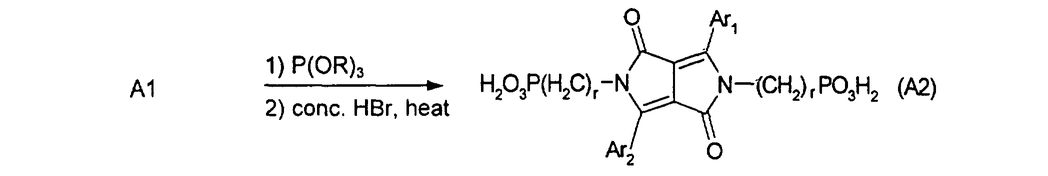 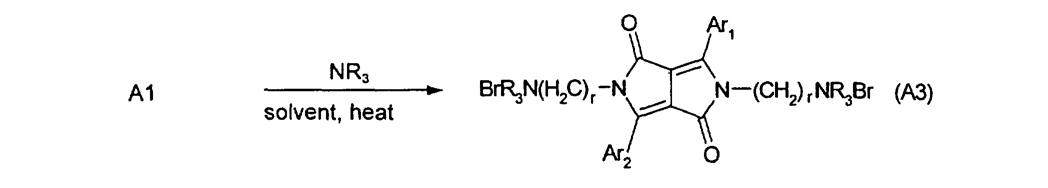 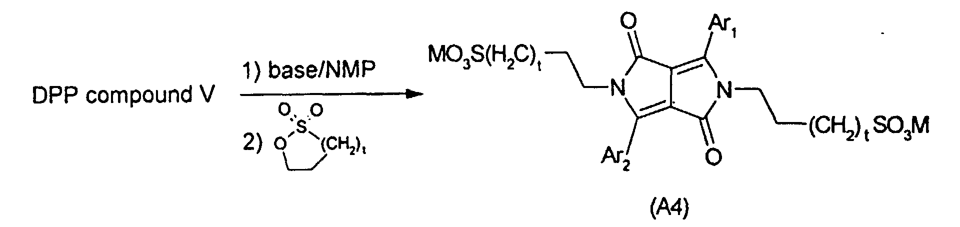 wherein M stands for a metal ion such as sodium or potassium, and t is 1 or 2

Accordingly, corresponding compounds III are available via such route.

The fluorescent diketopyrrolopyrroles of the formula (A2), (A3) or (A4) are new and represent a further embodiment of the present invention.
Accordingly, the present invention also relates to fluorescent diketopyrrolopyrroles of the formula (A2), (A3) or (A4) 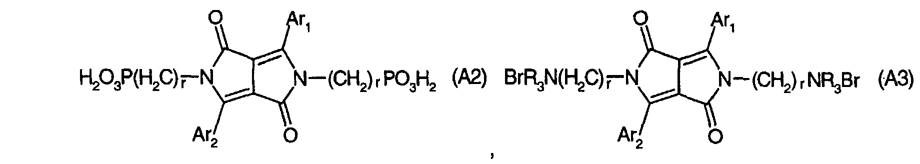 or 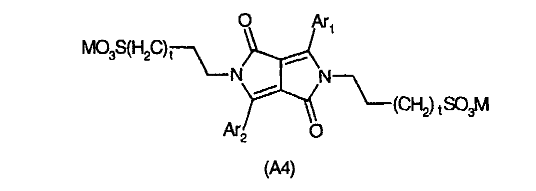 wherein r stands for an integer from 2 to 25; instead of linear alkyl groups, wherein the group -(CH₂)ᵣ- could be replaced by branched alkyl groups or aralkyl groups, such as -(CH₂)ᵣ₁-aryl-(CH₂)ᵣ₂-, r₁ and r₂ being whole numbers in the range of from 0 to 10; wherein M stands for a metal ion such as sodium or potassium, and t is 1 or 2,
Ar₁ and Ar₂, independently from each other, stand for aryl radicals, preferably for 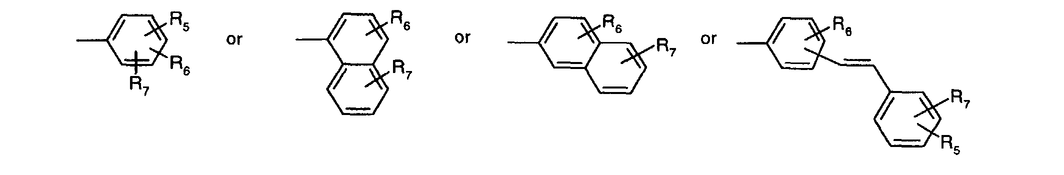 or 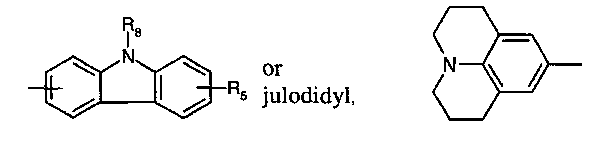 , which can be substituted one to four times with C₁-C₄alkyl, C₁-C₄alkoxy, or phenyl
or 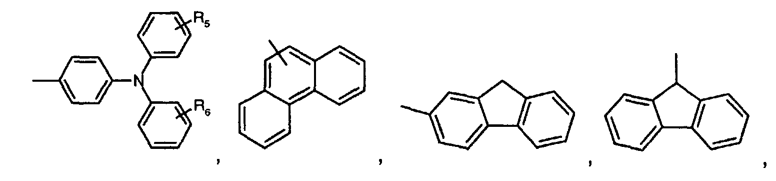 or 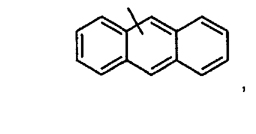 wherein
R₅, R₆ and R₇, independently from each other, stand for hydrogen, cyano, halogen, C₁-C₆alkyl, -NR₈R₉, -OR₁₀, -S(O)ₙR₈, -Se(O)ₙR₈, or phenyl, which can be substituted one to three times with C₁-C₈alkyl or C₁-C₈alkoxy,
wherein R₈ and R₉, independently from each other, stand for hydrogen, phenyl, C₁-C₂₅-alkyl, C₅-C₁₂-cycloalkyl, -CR₃R₄-(CH₂)ₘ-Ph, R₁₀, wherein R₁₀ stands for C₈-C₂₄-aryl, or a saturated or unsaturated heterocyclic radical comprising five to seven ring atoms, wherein the ring consists of carbon atoms and one to three hetero atoms selected from the group consisting of nitrogen, oxygen and sulfur, wherein Ph, the aryl and heterocyclic radical can be substituted one to three times with C₁-C₈alkyl, C₁-C₈alkoxy, or halogen, or
R₈ and R₉ stand for -C(O)R₁₁, wherein R₁₁ can be C₁-C₂₅-alkyl, C₅-C₁₂-cycloalkyl, R₁₀, -R₁₂ or -NR₁₃R₁₄, wherein R₁₂, R₁₃, and R₁₄ stand for C₁-C₂₅-alkyl, C₅-C₁₂-cycloalkyl, C₈-C₂₄-aryl, or a saturated or unsaturated heterocyclic radical comprising five to seven ring atoms, wherein the ring consists of carbon atoms and one to three hetero atoms selected from the group consisting of nitrogen, oxygen and sulfur, wherein the aryl and heterocyclic radical can be substituted one to three times with C₁-C₈alkyl or C₁-C₈alkoxy,
or -NR₈R₉ stands for a five- or sixmembered heterocyclic radical in which R₈ and R₉ together stand for tetramethylene, pentamethylene, -CH₂-CH₂-O-CH₂-CH₂-, or -CH₂-CH₂-NR₅-CH₂-CH₂-, preferably -CH₂-CH₂-O-CH₂-CH₂-, and n stands for 0, 1, 2 or 3.

Another embodiment of the present invention is related to a method of coloring high molecular weight organic materials (having a molecular weight usually in the range of from 10³ to 10⁷ g/mol) by incorporating the inventive fluorescent DPP compounds I or III by known methods in the art.

As high molecular weight organic materials the following can be used such as biopolymers, and plastic materials, including fibres.

The present invention relates preferably to the use of the inventive DPPs I or III for the preparation of
inks, for printing inks in printing processes, for flexographic printing, screen printing, packaging printing, security ink printing, intaglio printing or offset printing, for pre-press stages and for textile printing, for office, home applications or graphics applications, such as for paper goods, for example, for ballpoint pens, felt tips, fiber tips, card, wood, (wood) stains, metal, inking pads or inks for impact printing processes (with impact-pressure ink ribbons), for the preparation of
colorants, for coating materials, for industrial or commercial use, for textile decoration and industrial marking, for roller coatings or powder coatings or for automotive finishes, for high-solids (low-solvent), water-containing or metallic coating materials or for pigmented formulations for aqueous paints, for the preparation of
pigmented plastics for coatings, fibers, platters or mold carriers, for the preparation of
non-impact-printing material for digital printing, for the thermal wax transfer printing process, the ink jet printing process or for the thermal transfer printing process, and also for the preparation of
color filters, especially for visible light in the range from 400 to 700 nm, for liquid-crystal displays (LCDs) or charge combined devices (CCDs) or for the preparation of
cosmetics or for the preparation of
polymeric ink particles, toners, dry copy toners liquid copy toners, or electrophotographic toners.

Illustrative examples of suitable organic materials of high molecular weight which can be colored with the inventive fluorescent DPPs I or III of this invention are vinyl polymers, for example polystyrene, poly-a-methylstyrene, poly-p-methylstyrene, poly-p-hydroxystyrene, poly-p-hydroxyphenylstyrene, polymethyl methacrylate and polyacrylamide as well as the corresponding methacrylic compounds, polymethylmaleate, polyacrylonitrile, polymethacrylonitrile, polyvinyl chloride, polyvinyl fluoride, polyvinylidene chloride, polyvinylidene fluoride, polyvinyl acetate, polymethyl vinyl ether and polybutyl vinyl ether; polymers which are derived from maleinimide and/or maleic anhydride, such as copolymers of maleic anhydride with styrene; polyvinyl pyrrolidone; ABS; ASA; polyamides; polyimides; polyamidimides; polysulfones; polyether sulfones; polyphenylene oxides; polyurethanes; polyureas; polycarbonates; polyarylenes; polyarylene sulfides; polyepoxides; polyolefins such as polyethylene and polypropylene; polyalkadienes; biopolymers and the derivatives thereof e.g. cellulose, cellulose ethers and esters such as ethylcellulose, nitrocellulose, cellulose acetate and cellulose butyrate, starch, chitin, chitosan, gelatin, zein; natural resins; synthetic resins such as alkyd resins, acrylic resins, phenolic resins, epoxide resins, aminoformaldehyde resins such as urea/formaldehyde resins and melamine/formaldehyde resin; vulcanized rubber; casein; silicone and silicone resins; rubber, chlorinated rubber; and also polymers which are used, for example, as binders in paint systems, such as novolaks which are derived from C₁-C₆-aldehydes such as formaldehyde and acetaldehyde and a binuclear or mononuclear, preferably mononuclear, phenol which, if desired, is substituted by one or two C₁-C₉alkyl groups, one or two halogen atoms or one phenyl ring, such as o-, m- or p-cresol, xylene, p-tert.-butylphenol, o-, m- or p-nonylphenol, p-chlorophenol or p-phenylphenol, or a compound having more than one phenolic group such as resorcinol, bis(4-hydroxyphenyl)methane or 2,2-bis(4-hydroxyphenyl)propane; as well as suitable mixtures of said materials.

Particularly preferred high molecular weight organic materials, in particular for the preparation of a paint system, a printing ink or ink, are, for example, cellulose ethers and esters, e.g. ethylcellulose, nitrocellulose, cellulose acetate and cellulose butyrate, natural resins or synthetic resins (polymerization or condensation resins) such as aminoplasts, in particular urea/formaldehyde and melamine/formaldehyde resins, alkyd resins, phenolic plastics, polycarbonates, polyolefins, polystyrene, polyvinyl chloride, polyamides, polyurethanes, polyester, ABS, ASA, polyphenylene oxides, vulcanized rubber, casein, silicone and silicone resins as well as their possible mixtures with one another.

It is also possible to use high molecular weight organic materials in dissolved form as film formers, for example boiled linseed oil, nitrocellulose, alkyd resins, phenolic resins, melamine/formaldehyde and urea/formaldehyde resins as well as acrylic resins.

Said high molecular weight organic materials may be obtained singly or in admixture, for example in the form of granules, plastic materials, melts or in the form of solutions, in particular for the preparation of spinning solutions, paint systems, coating materials, inks or printing inks.

In a particularly preferred embodiment of this invention, the inventive fluorescent DPPs I or III are used for the mass coloration of polyvinyl chloride, polyamides and, especially, polyolefins such as polyethylene and polypropylene as well as for the preparation of paint systems, including powder coatings, inks, printing inks, color filters and coating colors.

Illustrative examples of preferred binders for paint systems are alkyd/melamine resin paints, acryl/melamine resin paints, cellulose acetate/cellulose butyrate paints and two-pack system lacquers based on acrylic resins which are crosslinkable with polyisocyanate.

According to observations made to date, the inventive fluorescent DPPs I or III can be added in any desired amount to the material to be colored, depending on the end use requirements. In the case of high molecular weight organic materials, for example, the fluorescent DPPs I or III prepared according to this invention can be used in an amount in the range from 0.01 to 40, preferably from 0.01 to 5% by weight, based on the total weight of the colored high molecular weight organic material.

Hence, another embodiment of the present invention relates to a composition comprising
(a) 0.01 to 50, preferably 0.01 to 5, particularly preferred 0.01 to 2% by weight, based on the total weight of the colored high molecular organic material, of a fluorescent DPP I or III according to the present invention, and
(b) 99.99 to 50, preferably 99.99 to 95, particularly preferred 99.99 to 98% by weight, based on the total weight of the colored high molecular organic material, of a high molecular organic material, and
(c) if desired, customary additives such as rheology improvers, dispersants, fillers, paint auxiliaries, siccatives, plasticizers, UV-stabilizers, and/or additional pigments or corresponding precursors in effective amounts, such as e.g. from 0 to 50% by weight, based on the total weight of (a) and (b).

To produce non-brittle mouldings or to diminish their brittleness, so-called plasticizers can be added to the high molecular weight organic materials prior to moulding. Plasticizers may be, for example, esters of phosphoric acid, phthalic acid and sebacic acid. Said plasticizers may be added before, during or after pigmenting the high molecular weight organic materials with the inventive fluorescent DPPs I or III.

To obtain different shades, the inventive fluorescent DPPs I or III may advantageously be used in admixture with fillers, transparent and opaque white, colored and/or black pigments as well as customary luster pigments in the desired amount.

For the preparation of paints systems, coating materials, color filters, inks and printing inks, the corresponding high molecular weight organic materials, such as binders, synthetic resin dispersions etc. and the inventive fluorescent DPPs I or III are usually dispersed or dissolved together, if desired together with customary additives such as dispersants, fillers, paint auxiliaries, siccatives, plasticizers and/or additional pigments or pigment precursors, in a common solvent or mixture of solvents. This can be achieved by dispersing or dissolving the individual components by themselves, or also several components together, and only then bringing all components together, or by adding everything together at once.

Hence, a further embodiment of the present invention relates to a method of using the inventive fluorescent DPPs I or III for the preparation of dispersions and the corresponding dispersions, and paint systems, coating materials, color filters, inks and printing inks comprising the inventive fluorescent DPPs I or III.

A particularly preferred embodiment relates to the use of the inventive DPPs I or III for the preparation of fluorescent tracers for e.g. leak detection of fluids such as lubricants, cooling systems etc., as well as to fluorescent tracers or lubricants comprising the inventive DPPs I or III. Usually, such lubricant compositions, e.g. for a refrigerant, comprise an-oil selected from the group consisting of naphthalenic oils, paraffinic oils, alkylated benzene oils, polyalkyl silicate oils, polyglycols, esters, polyether polyols, polyvinyl ethers, polycarbonates, fluorinated silicones, perfluoroethers, aromatic compounds with fluoroalkyloxy or fluoroalkylthio substituents. The amount of the inventive DPP I or III in the lubricant is chosen generally in an amount of from 100 to 1000 ppm. If the inventive compound I is water-soluble, it could be used as tracer in water as well.

A particular embodiment of this invention concerns ink jet inks comprising the inventive fluorescent compositions

The desired ink may contain up to 30 % by weight of the fluorescent composition, but will generally be in the range of 0.1 to 10, preferably from 0.1 to 8% by weight of the total ink composition for most thermal ink jet printing applications.

Further, the inks usually contain polymeric dispersants such as random, block, branched or graft polymers or copolymers. Most preferred are polymeric dispersants made by the group transfer polymerization process, because in general these are free from higher molecular weight species that tend to plug pen nozzles.

In AB or BAB block copolymers, the A segment usually is a hydrophobic homopolymer or copolymer which serves to link with the inventive fluorescent composition and the B block generally is a hydrophilic homopolymer or copolymer, or salts thereof and serves to disperse the pigment in the preferably chosen aqueous medium. Such polymeric dispersants and the synthesis thereof are known from e.g. US 5,085,698.

ABC triblocks are also useful as dispersants. In the ABC triblock, the A block usually is a polymer compatible with water, the B block is a polymer capable of binding to the fluorescent composition and the C block is compatible with the organic solvent. Preferably the A and C blocks are end blocks. ABC triblocks and their synthesis are disclosed e.g. in EP-A 556,649. Suitable graft polymers are disclosed in US 5,231,131.

Representative compounds useful for this purpose include e.g. polymers of polyvinyl alcohol, cellulosics and ethylene oxide modified polymers, and dispersant compounds containing ionisable groups such as acrylic acid, maleic acid or sulfonic acid.

The polymeric dispersant is generally present in an amount in the range of from 0.1 to 30, preferably from 0,1 to 8% by weight of the total ink composition.

In addition to, or in place of the preferred polymeric dispersants, surfactants may be used as dispersants. These may be anionic, nonionic, or amphoteric surfactants. A detailed list of non-polymeric as well as some polymeric dispersants is disclosed in the section on dispersants of Manufacturing Confection Publishing Co., (1990) p. 110-129, McCutcheon's Functional Materials, North America Edition.

Usually the ink contains an aqueous medium such as water or a mixture of water and at least one water-soluble organic solvent. Water-soluble organic solvents are well known, representative examples of which are disclosed in e.g. US 5,085,698. Selection of a suitable mixture of water and water-soluble organic solvent depends on usually requirements of the specific application such as desired surface tension and viscosity, drying time of the ink, and the media substrate onto which the ink will be printed.

Particularly preferred is a mixture of a water-soluble solvent having at least two hydroxyl groups, e.g. diethylene glycol, and water, especially deionized water.

In the event that a mixture of water and a water-soluble organic solvent is used as aqueous medium, water usually would comprise from 30 to 95, preferably 60 to 95% by weight, based on the total weight of the aqueous medium.

The amount of aqueous medium generally is in the range of from 70 to 99.8, preferably from 84 to 99.8%, based on the total weight of the ink.

The ink may contain other ingredients well known to those skilled in the art such as surfactants to alter surface tension as well as to maximize penetration. However, because surfactants may destabilize dispersions, care should be taken to insure compatibility of the surfactant with the other ink components. In general, in aqueous inks, the surfactants may be present in amounts ranging from 0.01 to 5, preferably from 0.2 to 3% by weight, based on the total weight of the ink.

Biocides may be used in the ink compositions to inhibit growth of microorganisms. Sequestering agents such as EDTA may also be included to eliminate deleterious effects of heavy metal impurities. Other known additives, such as viscosity modifiers may also be added.

A further embodiment concerns the use of the inventive fluorescent compounds I in phase change ink jet inks. The preparation of such inks is well known in the art, e.g. described in detail in EP-A 816, 410.

For the pigmentation of high molecular weight organic material, the inventive DPPs I or III, optionally in the form of masterbatches, usually are mixed with the high molecular weight organic materials using roll mills, mixing apparatus or grinding apparatus. Generally, the pigmented material is subsequently brought into the desired final form by conventional processes, such as calandering, compression molding, extrusion, spreading, casting or injection molding. In order to prepare non-rigid moldings or to reduce their brittleness it is often desired to incorporate so-called plasticizers into the high molecular weight organic materials prior to forming. Examples of compounds which can be used as such plasticizers are esters of phosphoric acid, phthalic acid or sebacic acid. The plasticizers can be added before or after the incorporation of the inventive DPPs I or III into the polymers. It is also possible, in order to achieve different hues, to add fillers or other coloring constituents such as white, color or black pigments in desired amounts to the high molecular weight organic materials in addition to the inventive DPPs I or III.

For pigmenting lacquers, coating materials and printing inks the high molecular weight organic materials and the inventive DPPs I or III, alone or together with additives, such as fillers, other pigments, siccatives or plasticizers, are generally dissolved or dispersed in a common organic solvent or solvent mixture. In this case it is possible to adopt a procedure whereby the individual components are dispersed or dissolved individually or else two or more are dispersed or dissolved together and only then are all of the components combined.

The present invention additionally relates to inks comprising a coloristically effective amount of the pigment dispersion of the inventive DPPs I or III.

Processes for producing inks especially for ink jet printing are generally known and are described for example in US 5,106,412.

The inks can be prepared, for example, by mixing the pigment dispersions comprising the inventive DPPs I or III with polymeric dispersants.

The mixing of the pigment dispersions with the polymeric dispersant takes place preferably in accordance with generally known methods of mixing, such as stirring or mechanical mixing; it is preferably advisable to use intensive mechanical mixers such as the so-called ULTRATURAX® stirrer from Kunkel & Jahn, Staufen (Germany).

When mixing a DPP I or III with polymeric dispersants it is preferred to use a water-dilutable organic solvent.

The weight ratio of the pigment dispersion to the ink in general is chosen in the range of from 0.001 to 75% by weight, preferably from 0.01 to 50% by weight, based on the overall weight of the ink.

Examples of suitable polymeric dispersants are carboxyl-containing polyacrylic resins such as polymeric methacrylic or crotonic acids, especially those obtained by addition polymerization of acrylic acid or acrylic acid and other acrylic monomers such as acrylates.

Depending on the field of use or when using DPP I or III, it is also possible, if desired, to admix a small proportion of a water-miscible organic solvent in from 0.01 to 30% by weight, based on the overall weight of the ink, and/or to admix water and/or bases so as to give a pH in the range from 7 to 11. It may likewise be advantageous to add preservatives, antifoams, surfactants, light stabilizers and pH regulators, for example, to the ink of the invention, depending on the field of use.

Examples of suitable pH regulators are inorganic salts such as lithium hydroxide or lithium carbonate, quaternary ammonium hydroxide or ammonium carbonate. Examples of preservatives and antifoams are, for example, sodium dehydroacetate, 2,2-dimethyl-6-acetoxydioxane or ammonium thioglycolate. It is also possible to employ known agents which regulate the viscosity or the surface tension and are described in e.g. US 5,085,698.

Examples of water-miscible organic solvents are aliphatic C₁-C₄alcohols, such as methanol, ethanol, n-propanol, isopropanol, n-butanol, tert.-butanol, ketones such as acetone methyl ethyl ketone, methyl isobutyl ketone or diacetone alcohol, and also polyols, Cellosolves® and carbitols, such as ethylene glycol, diethylene glycol, triethylene glycol, glycerol, propylene gylcol, ethylene glycol monomethyl or monoethyl ether, propylene glycol methyl ether, dipropylene glycol methyl ether, tripropylene glycol methyl ether, ethylene glycol phenyl ether, propylene glycol phenyl ether, diethylene glycol monomethyl or monoethyl ether, diethylene glycol monobutyl ether, triethylene glycol monomethyl or monoethyl ether, and also N-methyl-2-pyrrolidone, 2-pyrrolidone, N,N'-dimethylformamide or N,N'-dimethylacetamide.

If desired, the ink prepared as described above can be worked up further. The working up of the ink can be carried out by the customary methods for working up dispersions, by separation techniques, such as sieving or centrifuging the coarse particles from the resulting dispersion. It has been found advantageous, too, to carry out centrifuging in two stages of different intensity, e.g. centrifuging in a first step for from ten minutes to one hour at from 2000 to 4000 rpm and then, in a second step, for from 10 minutes to one hour at from 6000 to 10000 rpm.

Following centrifuging or sieving, the dispersion usually can be used directly as an ink for ink jet printing, for example.

The present invention additionally relates to a process for producing color filters comprising a transparent substrate and applied thereon a red, blue and green layer in any desired sequence, by using a red compound I and known blue and green compounds. The different colored layers preferably exhibit patterns such that over at least 5% of their respective surface they do not overlap and with very particular preference do not overlap at all.

The preparation and use of color filters or color-pigmented high molecular weight organic materials are well-known in the art and described e.g. in Displays 14/2, 1151 (1993), EP-A 784085, or GB-A 2,310,072.

The color filters can be coated for example using inks, especially printing inks, which can comprise pigment dispersions comprising the inventive DPPs I or III or can be prepared for example by mixing a pigment dispersion comprising a DPP I or III with chemically, thermally or photolytically structurable high molecular weight organic material (so-called resist). The subsequent preparation can be carried out, for example, in analogy to EP-A 654 711 by application to a substrate, such as a LCD, subsequent photostructuring and development.

Particular preference for the production of color filters is given to pigment dispersions comprising a DPP I or III which possess non-aqueous solvents or dispersion media for polymers.

The present invention relates, moreover, to toners comprising a pigment dispersion containing a DPP I or III or a high molecular weight organic material pigmented with a DPP I or III in a coloristically effective amount.

In a particular embodiment of the process of the invention, toners, coating materials, inks or colored plastics are prepared by processing masterbatches of toners, coating materials, inks or colored plastics in roll mills, mixing apparatus or grinding apparatus.

The present invention additionally relates to colorants, colored plastics, polymeric ink particles, or non-impact-printing material comprising an inventive DPP I or III pigment, preferably in the form of a dispersion, or a high molecular weight organic material pigmented with a DPP I or III in a coloristically effective amount.

A coloristically effective amount of the pigment dispersion according to this invention comprising an inventive DPP I or III denotes in general from 0.0001 to 99.99% by weight, preferably from 0.001 to 50% by weight and, with particular preference, from 0.01 to 50% by weight, based on the overall weight of the material pigmented therewith.

Further, the inventive compounds I can be used for textile application and for the dying of paper.

Another preferred embodiment concerns to the use of the inventive compounds for color changing media. There are three major techniques in order to realize full-color organic electroluminescent devices:
(i) to use the three primary colors blue, red and green by electroluminescence,
(ii) to convert electroluminescent blue to photoluminescent green and red via color changing media which absorb the above electroluminescent blue and fluoresce in green and red,
(iii) to convert white electroluminescent emission to blue, green and red via classical color filters.

The inventive compounds are useful for EL materials for the above category (i). In addition, the inventive compounds are also useful for the above technique (ii). This is because the invented compounds can exhibit strong photoluminescence as well as electroluminescence.

Technique (ii) is known for example from US 5,126,214 in which a method is described wherein EL blue with a maximum wavelength of ca. 480 nm to green, yellowish green, orange and red using coumarin, 4-(dicyanomethylene)-2-methyl-6-(p-dimethylaminostyryl)-4*H*-pyran, pyridine, rhodamine 6G, phenoxazone and other dyes.

In contrast to known red fluorescent dyes (thioindigos) the inventive DPPs I or III can be applied to color polyamides, because they do not decompose during the incorporation into the polyamides. Further, they exhibit an exceptionally good lightfastness, a superior heat stability, especially in plastics.

### Examples

The solid state absorbance spectra are measured on a Perkin-Elmer Lambda 9 UVNIS-spectrometer and the solid state fluorescence spectra on a Perkin-Elmer MPF 66 with a 5cm Ulbricht-sphere. The measurements are carried out with flexible PVC containing 0.02% by weight of the inventive compounds.

Preparation Example 1: Sodium hydride (60% dispersion in mineral oil, 47 g, 1.175 mol) is added portionwise over a 30-minutes period without external cooling and under nitrogen to a slurry of 1,4-diketo-3,6-bis-(4'-t-butylphenyl)pyrrolo[3,4-c]pyrrole (140 g, 0.33 mol) in 1-methyl-2-pyrrolidone (2 liters). After two hours the reaction mixture is cooled in an ice-water bath for 30 minutes, then benzyl bromide (216 g, 1.263 mol) is added dropwise (over 30 minutes). The reaction mixture is then slowly warmed-up to room temperature (by keeping the reaction flask in the cooling bath and allowing the ice in the bath to melt) and stirred at this temperature for 10 hours. Then acetic acid (50 ml), water (50 ml) and acetone (1.5 liters) are successively added. After stirring for 1 hour, a red solid is filtered off, washed with acetone (500 ml), water (4 liters), ethanol (1 liter), hexane (1 liter) and acetone (500 ml) and then dried under a reduced atmosphere at 50°C for 24 hours. Yield: 104 g (53%) of bright red solid 1,4-diketo-2,5-dibenzyl-3,6-bis-(4'-t-butylphenyl)pyrrolo[3,4-c]pyrrole.

Example 10:
(a) Triphenyl amine (98.32 g, 0.393 mol) is suspended in DMF (280 ml). Phosphorus oxychloride (66.24 g, 0.432 mol) is added dropwise to it over a 30-minutes period without external cooling. After stirring for one more hour, the reaction is heated to 80°C (bath temperature) for 2 ½ hours. After cooling to room temperature, the reaction is slowly poured onto ice-cold water (8 liters) with vigorous stirring. After 30 minutes, aqueous sodium hydroxide (5 N, 250 ml) is added to the reaction, and stirring is continued for one hour. The obtained precipitate is filtered off, washed with water (2 liters), then with methanol (2 litres) and dried to give 4-diphenylaminobenzaldehyde as a beige solid (90.47 g, 0.331 mol, 84 %), which is used in the next step without further purification.
(b) 48.7 g of the above obtained 4-diphenylaminobenzaldehyde (0.178 mol) is suspended in formic acid (400 ml). Hydroxylamine sulfate is added (16.08 g, 0.098 mol), followed by sodium formiate (14.15 g, 0.214 mol). The reaction is heated under reflux for 3 hours. Then the solvent is removed under vacuum. The residue is suspended in toluene (800 ml). The residual solid is filtered off and discarded. Solvents are evaporated and the residue is dissolved in a minimum amount of dichloromethane. This solution is filtered through a pad of silica gel, using dichloromethane as solvent. The solvent is then evaporated under vacuum. The thus obtained solid is taken up in toluene (350 ml) and heated to reflux in the presence of charcoal. After filtering hot, the solvent is removed from the filtrate to give 4-diphenylaminobenzonitrile (42.01 g, 0.155 mol, 87%) as a beige solid, which is taken to the next step without further purification.
(c) Sodium pieces (24.5 g, 1.064 mol) are added to tert.-amyl alcohol (400 ml). Then 20 mg of anhydrous FeCl₃ are added. The reaction mixture is slowly heated until a gentle reflux is obtained. After two hours, all sodium is reacted. 134.54 g of the above obtained 4-diphenylaminobenzonitrile (0.501 mol) are added in portions over a period of 15 minutes. Then di-tert.-butyl succinate (79.5 g, 0.346 mol) in tert.-amyl alcohol (300 ml) is added over 1.75 hours. After an additional hour of heating to reflux, the reaction mixture is cooled to room temperature and stirred overnight. Then, the reaction mixture is slowly added into a mixture of water (1200 ml) and methanol (600 ml) and stirred for 3 hours. The thus obtained solid is then filtered, washed with first water and then ethanol, and afterwards dried at 50°C overnight. 70.96 g (0.114 mol, 46%) of 1,4-diketo-3,6-bis-(4-diphenylaminophenyl)-pyrrolo[3,4-c]pyrrole are obtained as a violet powder. ¹H-NMR (300 MHz, d⁶-DMSO): 6.91 (d, 4 H, J = 9 Hz); 7.15-7.22 (m, 12 H); 7.38-7.43 (m, 8 H); 8.32 (d, 4 H, J = 9 Hz); 11.02 (broad s, 2 H).
(d) Preparation Example 1 is repeated except that the above obtained 1,4-diketo-3,6-bis-(4-diphenylaminophenyl)pyrrolo[3,4-c]pyrrol is used. Yield: 56%.

Preparation Example 18: Example 13 is repeated except that 3,5-dimethylbenzyl bromide is used as alkylating agent. Red solid (Yield: 24%).

Example 22: 24.6 g of potassium tert.-butoxide, 30 g of 2-naphthonitrile and 200 ml of tert.-amyl alcohol are heated up to 100°C under a nitrogen atmosphere. As soon as the this temperature has been reached, a solution of 23 g of di-n-butyl succinate and 70 ml of tert.-amyl alcohol is added over 1 hour using a dropping funnel. When the addition is completed, the reaction mixture is kept for 16 hours at 100°C, then cooled to 65°C, neutralized with 20 ml of glacial acetic acid and boiled briefly to reflux. The resultant pigment suspension is filtered at room temperature. The filter cake is suspended in 300 ml of methanol and the pigment is isolated again by filtration, then finally washed with methanol and water until washings run colourless, and dried at 100°C in an atmosphere under reduced pressure affording 26.1 g (69% of theory, based on dibutyl succinate) of pure pigment of 1,4-diketo-3,6-bis-(2-naphthyl)-pyrrolo-(3,4-c)-pyrrole.
And then, Preparation Example 18 is repeated except that 1,4-diketo-3,6-bis-(2-naphthyl)-pyrrolo-(3,4-c)-pyrrole is used as starting material. Red solid (Yield: 36%).

Example 23: Example 22 is repeated except that benzyl bromide is used as alkylating agent. Orange solid (Yield: 30 %).

Example 24: Example 22 is repeated except that 2-methylbenzyl bromide is used as alkylating agent. Orange solid (Yield: 30 %).

Example 25: Example 22 is repeated except that 2-phenylbenzyl bromide is used as alkylating agent. Red solid (Yield: 8 %).

Example 29: Example 22 is repeated except that 3-methylbenzyl bromide and 1,4-diketo-3,6-bis-(2-naphthyl)-pyrrolo-(3,4-c)-pyrrole are used as alkylating agent and starting material, respectively. Red solid (Yield: 30%).

Example 30: And then, Example 29 is repeated except that 4-methylbenzyl bromide is used as alkylating agent. Red solid (Yield: 36%).

Example 31: Example 29 is repeated except that 4-phenylbenzyl bromide is used as alkylating agent. Orange solid (Yield: 30 %).

Example 35: 9.2 g of potassium tert.-butoxide, 15 g of 6-methoxy-2-naphthonitrile and 80 ml of tert.-amyl alcohol are heated up to 100°C under a nitrogen atmosphere. As soon as the this temperature is reached, a solution of 9.4 g of di-n-butyl succinate and 20 ml of tert.-amyl alcohol is added over 1 hour using a dropping funnel. When the addition is completed, the reaction mixture is kept for 12 hours at 100°C, then cooled to 65°C, neutralized with 20 ml of glacial acetic acid and boiled briefly to reflux. The resultant pigment suspension is filtered at room temperature. The filter cake is suspended in 300 ml of methanol and the pigment is isolated again by filtration, then finally washed with methanol and water until washings run colourless, and dried at 100°C in an atmosphere under reduced pressure which affords 4.2 g (23% of theory, based on dibutyl succinate) of 1,4-diketo-3,6-bis-(2-(6-methoxynaphthyl))-pyrrolo-(3,4-c)-pyrrole.
Example 29 is repeated except that 1,4-diketo-3,6-bis-(6-methoxy-2-naphthyl)-pyrrolo-(3,4-c)-pyrrole are used as starting material. Yellow solid (Yield: 21 %).

Example 36: Example 35 is repeated except that 3,5-dimethylbenzyl bromide is used as alkylating agent. Yellow solid (Yield: 38%).

Preparation Example 37: 2.2 g (5.0 mmol) 1,4-diketo-3,6-bis-(4-biphenyl)-pyrrolo-(3,4-c)-pyrrole are slurred in 20 ml of 1-methyl-2-pyrrrolidinone for 2 hours at room temperature. 1.4 g (13.0 mmol) of potassium t-butoxide are added to the slurry under nitrogen. After stirring for 2hours, 2.78 g (12 mmol) of (2-iodoethyl) benzene are added to the reaction mixture. The mixture is heated up to 80°C and stirred additionally for 3 hours. After cooling to room temperature, the mixture is poured into 50 ml of water and a red solid is filtered off and purified by column chromatography (silica gel, dichloromethane as eluent). After drying, 0.16 g (5%) of a red solid are obtained.

Example 38: Preparation Example 37 is repeated except that 1,4-diketo-3,6-bis-(2-naphthyl)-pyrrolo-(3,4-c)-pyrrole is used as starting material (Yield: 29%).

Example 42: Example 29 is repeated except that 3-phenylbenzyl bromide is used as alkylating agent. Orange solid (Yield: 35%).

Example 45: Example 29 is repeated except that 3-methoxybenzyl bromide is used as alkylating agent. Orange solid (Yield: 30 %).

Preparation Example 52: 1.87 g (5.0 mmol) 1,4-diketo-3,6-bis-(4-dimethylaminophenyl)-pyrrolo-(3,4-c)-pyrrole are slurred in 60 ml of 1-methyl-2-pyrrrolidinone for 2 hours at room temperature. 1.68 g (15.0 mmol) of potassium tert.-butoxide are added to the slurry under nitrogen. After stirring for 2 hours, 2.78 g (15 mmol) of 1-bromoethyl benzene are added to the reaction mixture. The mixture is then heated up to 80°C and stirred additionally for 2 hours. After cooling to room temperature, the mixture is poured into 50 ml of water and a red solid is filtered off and purified by column chromatography (silica gel, dichloromethane as eluent). After drying, 0.2 g (10%) of a red solid are obtained.

Preparation Example 53: Example 52 is repeated except that 3,5-di-t-butylbenzyl bromide as alkylating agent. Red solid ( Yield : 33%).

Example 55. Preparation Example 53 is repeated except that 1,4-diketo-3,6-bis-(6-methoxy-2-naphthyl)-pyrrolo-(3,4-c)-pyrrole is used as starting material. Red solid (Yield : 21 %).

Example 57: Preparation Example 53 is repeated except that 1,4-diketo-3,6-bis-(2-naphthyl)-pyrrolo-(3,4-c)-pyrrole as starting material. Red solid ( Yield : 23%).

Example 59: 6.7 g ( 60 mmol) of potassium tert.-butoxide, 10.7 g ( 52 mmol) of 4-cyano-trans-stilbene and 100 ml of tert.-amyl alcohol are heated up to 100°C under a nitrogen atmosphere. As soon as the temperature is reached, a solution of 5.98g (26 mmol) of di-n-butyl succinate and 50 ml of tert.-amyl alcohol is added over 1 hour using a dropping funnel. When the addition is completed, the reaction mixture is kept for 16 hours at 100°C, then cooled down to 65°C, neutralized with 20 ml of glacial acetic acid and boiled briefly to reflux temperature. The resultant pigment suspension is filtered at room temperature. The filter cake is suspended in 100 ml of methanol and the pigment is isolated by filtration, then finally washed with methanol and water until washings run colourless, and dried at 100°C in an atmosphere under reduced pressure which affords 2.5 g (20% of theory, based on dibutyl succinate) of pure pigment of 1,4-diketo-3,6-bis-(4-trans-stilbene)-pyrrolo-(3,4-c)-pyrrole. Example 53 is repeated except that and 1,4-diketo-3,6-bis-(4-trans-stilbene)-pyrrolo-(3,4-c)-pyrrole is used as starting material. Red solid (Yield: 20%).

Example 60: Example 59 is repeated except that 3,5-dimethylbenzyl bromide as alkylating agent. Red solid (Yield : 33%).

Example 63: Example 53 is repeated except that 1,4-diketo-3,6-bis-(4-cis-stilbene)-pyrrolo-(3,4-c)pyrrole is used as starting material. Red solid (Yield : 36%).

Example 64: Example 36 is repeated except that 3-phenylbenzyl bromide as alkylating agent. Red solid (Yield : 25%).

Example 68: To the below mentioned engineering plastics (each 400 g) in chip form the inventive compounds (each 0.12 g) are added in a paint shaker and shaken there for 90 seconds. Thereafter, the thus obtained chips comprising the adhered inventive compounds are molded at the temperatures specified in Table 1 below using a BA400 Battenfeld injection molder.

**Table 1**

| Engineering plastic | Temperature settings (°C) | | |
|---|---|---|---|
| | Nozzle | Middle | Rear |
| HIPS | 232 (450°F) | 232 (450°F) | 232 (450°F) |
| ABS | 232 (450°F) | 232 (450°F) | 232 (450°F) |
| Nylon 6,12 | 271 (520°F) | 260 (500°F) | 254 (490°F) |
| PMMA | 226 (440°F) | 226 (440°F) | 226 (440°F) |

HIPS : high impact polystyrene (825P1 from Fina Oil and Chemical; melt flow (g/10 min): 8 (reference ASTM TEST 200/5.0 D-1238))
ABS : acrylic-butadiene-styrene copolymer (Natural ABS 3501-002 from Diamond Polymer; melt flow (g/10 min): 7.5 (reference ASTM Method D-1238))
Nylon 6,12 : polyamide (ZYTEL®158L from DuPont Engineering Polymers; inherent viscosity: 1.15))
PMMA: polymethylmethacrylate (PLEXIGLAS®V825 from Atohaas; melt flow (g/10 min): 3.7 (reference ASTM Method D-1238))

Five chips (from the same series) obtained from the injection molder are collected after the color is distributed homogeneously.

The color chips are then mounted, with the thickest part of the chip exposed (0.31 cm (0.122 inches)), in a Cl35A Atlas Xenon Weather-O-Meter. The parameters of the weather-o-meter are listed in Table 2.

The color chips are then exposed to the weather-o-meter for 100, 250, 500, 750, and 1000 hours. The color chips are rated after each fading interval is reached. The lightfastness is subjectively evaluated using a gray scale rating of 1-5. A rating of 5 indicates no fade or color difference. In cases where the color chip appears to darken a rating of d for darkening is applied to the gray scale rating. In cases where the color chip loses most of its color a rating of f for fading is applied to the gray scale rating.

**Table 2:**

| Parameters for the fading test | |
|---|---|
| Automatic voltage 0.35 W/m² at 340 nm | |
| | Light Cycle Settings |
| | Temperature [°C] |
| Black Panel¹⁾ | 63 |
| Wet Bulb Depression²⁾ | 10 |
| Conditioning Water³⁾ | 30 |

automatic voltage: controls irradiance level (similar to the average irradiance found on a clear summer day in southern Florida)
1) temperature is measured by a sensor attached to the specimen holder; provides a temperature reading resulting from the chamber air and any heating due to the xenon light
2) difference between air temperature (measured by the so-called dry bulb sensor) and wet bulb temperature (measured by wet bulb sensor, whereby the wet bulb is covered by a moistened wick; due to the cooling effect of evaporation, the wet bulb reading is usually lower than the dry bulb reading (except at 100% relative humidity).

**Table 6:**

| HIPS evaluation | | | | | |
|---|---|---|---|---|---|
| used DPP of example no. | Lightfastness evaluation using the gray scale | | | | |
| | 100 h | 250 h | 500 h | 700 h | 1000 h |
| 10d | 4/5 | 4 | 4d | 3d | 3d |
| Vat Red 41 | 3 | 2/3 | 1f | | |

Example 69: On an ITO glass substrate (from Geomatech Co. Ltd., ITO film thickness 200 nm, sheet resistance 10 Ω/cm²), the TPD compound of the following formula 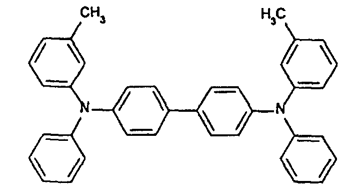 is deposited as a hole transporting substance by vacuum evaporation under a reduced pressure of 6.665x10⁻⁴ Pa (5.0x10⁻⁶ Torr) and at a depositing rate of 0.05 nm/sec to a membrane thickness of 50 nm to form a hole transporting layer. Then, on the hole transporting layer thus prepared, the compound of Example 22 is deposited as a light-emitting material under a depositing condition of 6.665x10⁻⁴ Pa (5.0x10⁻⁶ Torr) and 0.05 nm/sec to a membrane thickness of 50 nm to form a light-emitting layer. Then, on the light-emitting layer, firstly lithium is co-deposited with the above compound at a rate of 0.015 nm/sec to form a 1 nm-thick layer and subsequently aluminum as cathode are deposited on it to a film thickness of 200 nm. By using the ITO side as the anode and the aluminum side as the cathode, a bias of 20 V is applied to the above element. A luminescence showing a luminance of 1410 cd/m² with the EL emission peak wavelength at 560 nm is confirmed as the average value of the five elements. For evaluation of luminance and emission spectrum, the Luminometer BM-8 manufactured by TOPCON Co, Ltd. and the Multichannel Photodetector IMUC-7000 manufactured by Otsuka Electronics are used, respectively.

Example 70 - 101: Example 69 is repeated replacing the light-emitting material with the compounds indicated in Table 7 below, summarizing the EL performances also (the results of Example 69 is included also in the table).

**Table 7**

| Example | Light-emitting Material (Example) | EL Emission-Peak Wavelength (nm) | EL Intensity (cd/m²) |
|---|---|---|---|
| 84 | 22 | 579 | 1303 |
| 85 | 23 | 579 | 3970 |
| 86 | 24 | 585 | 3340 |
| 87 | 25 | 584 | 430 |
| 89 | 29 | 578 | 2810 |
| 90 | 30 | 577 | 1473 |
| 91 | 31 | 579,619 | 27 |
| 94 | 35 | 585 | 1842 |
| 99 | 45 | 575 | 1334 |

Example 104: 28 g (0.25 mol) of potassium tert.-butoxide, 62.5g (0.23 mol) of 4-(diphenylamino)benzonitrile and 300 ml of tert.-amyl alcohol are heated up to 100°C under a nitrogen atmosphere. As soon as the this temperature has been reached, a solution of 26.7 g (0.12 mol) of di-n-butyl succinate and 70 ml of tert.-amyl alcohol is added over 1 hour using a dropping funnel. When the addition is completed, the reaction mixture is kept for 16 hours at 100°C, then cooled down to 65°C, neutralized with 20 ml of glacial acetic acid and boiled briefly at reflux temperature. The resultant pigment suspension is filtered at room temperature. The filter cake is suspended in 300 ml of methanol and the pigment is isolated again by filtration, then finally washed with methanol and water until washings run colourless, and dried at 100°C in vacuo, affording 10.8 g (15% of theory, based on dibutyl succinate) of pure pigment of 1,4-diketo-3,6-bis-(4-diphenylaminophenyl)-pyrrolo-(3,4-c)-pyrrole.
1.02 g (1.64 mmol) 1,4-diketo-3,6-bis-(4-diphenylaminophenyl)-pyrrolo-(3,4-c)-pyrrole are slurred in 20 ml of 1-methyl-2-pyrrrolidinone for 2 hours at room temperature. 0.57g (5.09 mmol) of potassium tert.-butoxide is added to the slurry under nitrogen. After stirring for 2 hours, 1.37 g (4.84 mmol) of 3,5di-t-butyllbenzyl bromide are added to the reaction mixture and then the mixture is stirred additionally for 2 hours. After the reaction mixture is poured into 50 ml of water, the obtained solid is filtered off and purified by column chromatography (silica gel, dichloromethane as eluent). After drying, 0.444 g (26%) of a red solid are obtained.

The compound obtained above exhibits the optical properties summarized in the table below:

| in toluene | | | | in evaporated film | |
|---|---|---|---|---|---|
| λₘₐₓ^{*1} nm | ε^{*2} | Fₘₐₓ^{*3} nm | Φ^{*4} | λₘₐₓ^{*1} nm | Fₘₐₓ^{*3} nm |
| 533 | 47400 | 591 | 0.48 | 537 | 609 |

| | | | | | |
|---|---|---|---|---|---|
| *1: wavelength at optical absorption maximum, | | | | | |
| *2: molar absorption coeffcient | | | | | |
| *3: wavelength at photoluminescence maximum, | | | | | |
| *4: FQY | | | | | |

Example 103 is then repeated for EL device preparation replacing the light-emitting material with the film co-deposited using tris-(8-hydroxyquinolinato)aluminum(III) (manufactured by Wako Pure Chemicals Industries, Ltd.) and the compound prepared above (0.50 wt.-%). The co-deposition is done under a depositing condition of 6.665x10⁻⁴ Pa (5.0x10⁻⁶ Torr) and 300 pm/s (3.0 Å/s) for the aluminum complex and 1.5 pm/s (0.015 Å/s) for the above compound. For comparison, the device employing the aluminum complex for light-emitting substance is prepared.

The device using a light-emitting layer comprising of solely the aluminum complex begins to emit a green EL emission starting at 8 V. The emission maximum is at 520 nm with the intensity 6980 cd/m² at 25 V. The device using a light-emitting layer comprising the complex and the compound begins to emit from at 4 V (see the table below for the device performance). The wavelength of the EL emission maximum is at 590 nm, i.e. orange emission which is different from that of the single component device above. This suggests that the emission is induced via resonance energy transfer from the aluminum complex to the compound invented.

| Bias voltage (V) | EL Intensity (cd/m²) |
|---|---|
| 15 | 540 |
| 16 | 1300 |
| 17 | 3900 |
| 18 | 12520 |

The above results demonstrate that the invented compounds are useful for energy acceptor of Host-Guest type of light-emitting materials.

Preparation Example 105: 2.09 g (4.75 mmol) 1,4-diketo-3,6-bis-(4-biphenyl)-pyrrolo-(3,4-c)-pyrrole are slurred in 30 ml of 1-methyl-2-pyrrolidinone for 2 hours at room temperature. 1.29 g (11.52 mmol) of potassium tert.-butoxide are added to the slurry under an atmosphere of nitrogen. After stirring for 1 hour, 1.21 g (10 mmol) of allyl bromide are added to the reaction mixture and then the mixture is stirred additionally for two hours. The mixture then is poured into 50 ml of water and the obtained red solid is filtered off and then purified by column chromatography (silica gel, dichloromethane as eluent). After drying, 1.13 g (60%) of a red solid are obtained.

Example 109: A mixture of 45 g (0.4 mol) of potassium tert.-butoxide, 82 g (0.373 mol) of 9-ethyl-3-cyanocarbazole and 300 ml of tert.-amylalcohol is heated up to a temperature of 110°C under a nitrogen atmosphere. As soon as this temperature is reached, a solution of 43 g (0.18 mol) of di-n-butyl succinate and 100 ml of tert.-amyl alcohol are added over 1.5 hours using a dropping funnel. When the addition is complete, the reaction mixture is kept for 16 hours at 110°C, then cooled to 65°C, neutralized with 40 ml glacial acetic acid and boiled briefly at reflux temperature. The resultant pigment suspension is filtered at room temperature. The filter cake is suspended in 300 ml of methanol and the pigment is isolated by filtration, then finally washed with methanol and water until washings run colorless, and dried at 100°C under an atmosphere of reduced pressure. 10.5 g (11%) of pure 1,4-diketo-3,6-bis-(3-9-ethylcarbazole)-pyrrolo-(3,4-c)pyrrole are obtained.

Example 110: Preparation Example 105 is repeated except that 1,4-diketo-3,6-bis-(2-napthyl)-pyrrolo-(3,4-c)-pyrrole and 3,5-di-tert.-butylbenzylbromide are used. A red solid (36%) is obtained.

Example 111: Example 110 is repeated except that 3,5-dimethylbenzyl bromide is used instead of 3,5-di-tert.-butylbenzylbromide. A red solid (30%) is obtained.

Example 112: Example 109 is repeated except that 1-(4-cyanophenyl)-2-(3,5-di-tert.-butylphenyl)-trans-ethylene is used instead of 9-ethyl-3-cyanocarbazole. A red solid (5%) is obtained.

Example 113: Example 112 is repeated except that 3,5-dimethylbenzyl bromide is used as alkylating agent. A red solid (8%) is obtained.

Example 114: Example 10 is repeated except that 3-bromo benzyl bromide as alkylating agent. A red solid (23%) is obtained.

Example 115: Example 10 is repeated except that methyl iodide is used as alkylating agent. A red solid (40%) is obtained.

Example 116: Example 10 is repeated except that 3-methylbenzyl bromide is used as alkylating agent. A red solid (45%) is obtained.

Examples 119 to 129: Example 104 is repeated replacing only the guest material in the binary system with the compounds listed in Table 9 below, summarizing the EL performances also:

**Table 9**

| Example | Guest Material (example) | Guest Concentration [wt.-%] to Alq₃ | EL Emission Peak Wavelength [nm] | EL Intensity [cd/m²] |
|---|---|---|---|---|
| 119 | 57 | 1.4 | 555 | 4820 |
| 121 | 55 | 1.3 | 566 | 7329 |
| 122 | 59 | 1.8 | 591 | 5344 |
| 123 | 104 | 1.3 | 608 | 11004 |
| 124 | 114 | 1.5 | 619 | 9831 |
| 125 | 109 | 1.3 | 567 | 8745 |
| 126 | 112 | 1.8 | 591 | 5302 |
| 127 | 115 | 1.4 | 611 | 6398 |
| 128 | 116 | 2 | 617 | 8784 |
| 129 | 10 | 2 | 621 | 7108 |

Example 130 to 134: Example 69 is repeated replacing the light emitting material with the compounds listed in Table 10 below, summarizing the EL performances also:

**Table 10**

| Example | Guest Material (example) | EL Emission Peak Wavelength [nm] | EL Intensity [cd/m²] |
|---|---|---|---|
| 132 | 109 | 588, 625 | 2330 |
| 133 | 112 | 605 | 1654 |
| 134 | 113 | 624 | 875 |

Example 135: 294 mg of in tetrahydrofurane recrystallized Alq₃ (manufactured from Tokyo Kasei Organic Chemicals) and 6 mg of 1,4-diketo-2,5-bis-(3,5-di-tert.-butylbenzyl)-3,6-bis-(4-diphenylaminophenyl)-pyrrolo-(3,4-c)pyrrole are dissolved in 50 ml of dichloromethane. The thus obtained solution is slowly poured into 500 ml of hot water, and the thus obtained precipitate is collected and dried. The obtained precipitate is purified by means of sublimation with heating under an atmosphere of reduced pressure of 6.65·10⁻⁴ Pa (5·10⁻⁶ Torr). 250 mg (5%) of a red host/guest composite are obtained.

Example 103 is then repeated for EL device preparation replacing the light-emitting material with the above obtained host/guest composite.

Examples 136 to 138: Example 135 is repeated without a replacement of the guest concentration listed in Table 11 below:

**Table 11**

| Example | Guest Concentration [wt.-%] | EL Emission Peak Wavelength [nm] | EL Intensity [cd/m²] |
|---|---|---|---|
| 136 | 5 | 615 | 9013 |
| 137 | 3 | 617 | 10085 |
| 138 | 2 | 613 | 9686 |

## Claims

1. Electroluminescent device comprising in this order
(a) an anode
(b) a hole transporting layer
(c) a light-emitting layer
(d) optionally an electron transporting layer and
(e) a cathode
and a light-emitting substance, wherein the light-emitting substance is a diketopyrrolopyrrole ("DPP") represented by formula I or formula III 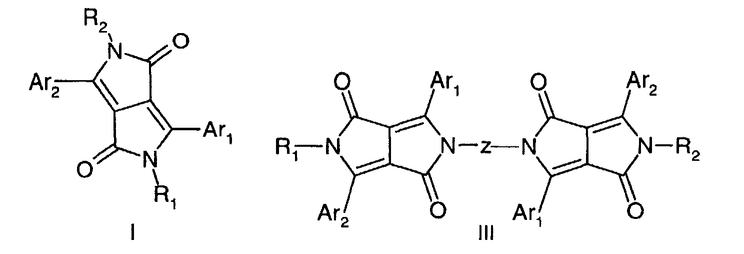 wherein R₁ and R₂, independently from each other, stand for C₁-C₂₅-alkyl, allyl which can be substituted one to three times with C₁-C₃alkyl or Ar₃, or -CR₃R₄-(CH₂)ₘ-Ar₃, wherein R₃ and R₄ independently from each other stand for hydrogen or C₁-C₄alkyl, or phenyl which can be substituted one to three times with C₁-C₃ alkyl,
Ar₃ stands for phenyl or 1- or 2-naphthyl which can be substituted one to three times with C₁-C₈alkyl, C₁-C₈alkoxy, halogen or phenyl, which can be substituted with C₁-C₈alkyl or C₁-C₈alkoxy one to three times, and m stands for 0, 1, 2, 3 or 4,
Ar₁ and Ar₂, independently from each other, stand for 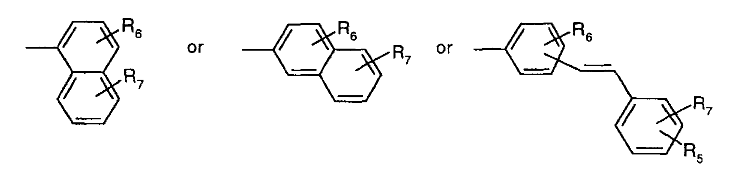 or 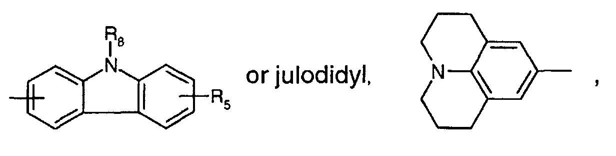 which can be substituted one to four times with C₁-C₄alkyl, C₁-C₄alkoxy, or phenyl or 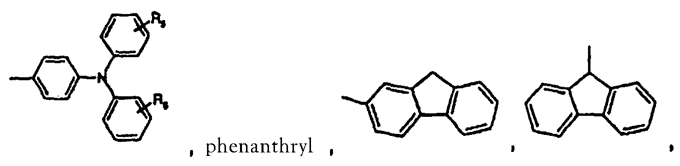 or 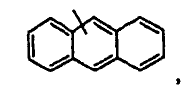 wherein
R₅, R₆ and R₇, independently from each other, stand for hydrogen, cyano, halogen, C₁-C₆alkyl, -NR₈R₉, -OR₁₀, -S(O)ₙR₈, -Se(O)ₙR₈, or phenyl, which can be substituted one to three times with C₁-C₈alkyl or C₁-C₈alkoxy,
wherein R₈ and R₉, independently from each other, stand for hydrogen, phenyl, C₁-C₂₅-alkyl, C₅-C₁₂-cycloalkyl, -CR₃R₄-(CH₂)ₘ-Ph, R₁₀, wherein R₁₀ stands for C₈-C₂₄-aryl, or a saturated or unsaturated heterocyclic radical comprising five to seven ring atoms,
wherein the ring consists of carbon atoms and one to three hetero atoms selected from the group consisting of nitrogen, oxygen and sulfur, wherein Ph, the aryl and heterocyclic radical can be substituted one to three times with C₁-C₈alkyl, C₁-C₈alkoxy, or halogen, or R₈ and R₉ stand for -C(O)R₁₁, wherein R₁₁ can be C₁-C₂₅-alkyl, C₅-C₁₂-cycloalkyl, R₁₀, -OR₁₂ or -NR₁₃R₁₄, wherein R₁₂, R₁₃, and R₁₄ stand for C₁-C₂₅-alkyl, C₅-C₁₂-cycloalkyl, C₈-C₂₄-aryl, or
a saturated or unsaturated heterocyclic radical comprising five to seven ring atoms, wherein the ring consists of carbon atoms and one to three hetero atoms selected from the group consisting of nitrogen, oxygen and sulfur, wherein the aryl and heterocyclic radical can be substituted one to three times with C₁-C₈alkyl or C₁-C₈alkoxy, or -NR₈R₉ stands for a five- or sixmembered heterocyclic radical in which R₈ and R₉ together stand for tetramethylene, pentamethylene, -CH₂-CH₂-O-CH₂-CH₂-, or -CH₂-CH₂-NR₅-CH₂-CH₂-, preferably -CH₂-CH₂-O-CH₂-CH₂-, and n stands for 0, 1, 2 or 3, and wherein Z stands for a diradical selected from the group consisting of a single bond, C₂-C₆alkylene, which can be substituted one to three times with C₁-C₄alkyl, C₁-C₄alkoxy, or phenyl, phenylene or naphthylene.

2. Process for the preparation of compounds I or III according to claim 1 in treating in a first step the DPP derivative of formula Va or formula Vb 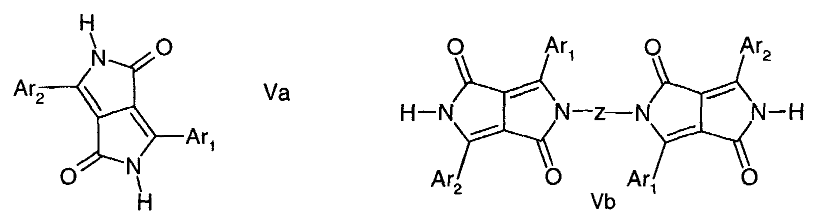 wherein Ar₁ and Ar₂ are defined as in claim 1, with a base, then, in a second step, treating the reaction mixture obtained in the first step with an alkylating agent, wherein in the first step the base is a hydride, an alkali metal alkoxide or a carbonate, and the alkylating agent is a sulfonate, tosylate, mesylate, carbonate, sulfate, or halogen compound of the formula (R₁)_{1 or 2}X, wherein X stands for SO₃-, (p-Me-phenyl)SO₂-, (2,4,6-trimethyl-phenyl)-SO₂-, -CO₃-, -SO₄-, or halogen, or a mixture of (R₁)_{1 or 2}X and (R₂)_{1 or 2}X.

3. Process for the preparation of compounds I according to claim 1 treating the DPP derivative of formula VIa or formula Vlb 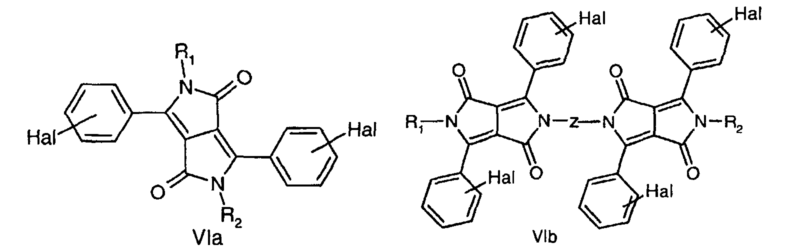 with a nucleophilic agent such as a secondary amine, HNR₈R₉, a thiol, HSR₈, or HS(O)ₙR₈, an alcohol, HOR₁₀, a diselenide, R₈(O)ₙSe-Se(O)ₙR₈, preferably in a molar ratio of DPP VIa or VIb:nucleophilic agent in the range of 1.2:1 to 0.8:1, or, if R₂ has the same meaning as R₁ in the range of from 1:2.5 to 1:1, in the presence of an anhydrous dipolar aprotic solvent, and of an anhydrous base in an amount in the range of from usually 0.1 to 15 moles per mole of the nucleophilic agent, at a temperature in the range of from 100 to 220°C and under a pressure in the range of from 100 to 300 kPa, and optionally isolating the obtained compound Va, resp. Vb, wherein R₁, R₂, R₈, R₉ and R₁₀ are as defined in claim 7 and Hal stands for halogen.

4. Method of coloring high molecular weight organic materials by incorporating the DPP compounds I or III according to claim 1 into said materials in an analogous matter to known methods.

5. Composition comprising
(a) 0.01 to 50% by weight, based on the total weight of the colored high molecular weight organic material, of a fluorescent DPP I or III according to claim 1, and
(b) 99.99 to 50% by weight, based on the total weight of the colored high molecular weight organic material, of a high molecular organic material, and
(c) 0 to 50% by weight customary additives.

6. Composition according to claim 5, wherein the high molecular weight organic material is a polyamide, a polystyrene, preferably high impact polystyrene, polymethylmethacrylate or an ABS copolymer.

7. Fluorescent diketopyrrolopyrroles represented by formula I or formula III 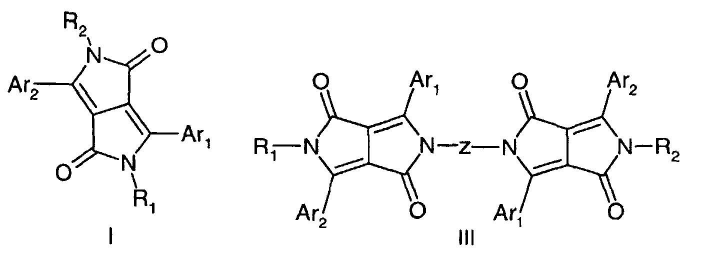 wherein R₁ and R₂, independently from each other, stand for C₁-C₂₅-alkyl, allyl which can be substituted one to three times with C₁-C₃alkyl or Ar₃, or -CR₃R₄-(CH₂)ₘ-Ar₃, wherein R₃ and R₄ independently from each other stand for hydrogen or C₁-C₄alkyl, or phenyl which can be substituted one to three times with C₁-C₃ alkyl,
Ar₃ stands for phenyl or 1- or 2-naphthyl which can be substituted one to three times with C₁-C₈alkyl, C₁-C₈alkoxy, halogen or phenyl, which can be substituted with C₁-C₈alkyl or C₁-C₈alkoxy one to three times, and m stands for 0, 1, 2, 3 or 4,
Ar₁ and Ar₂, independently from each other, stand for 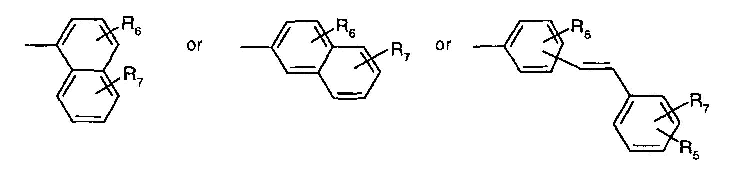 or 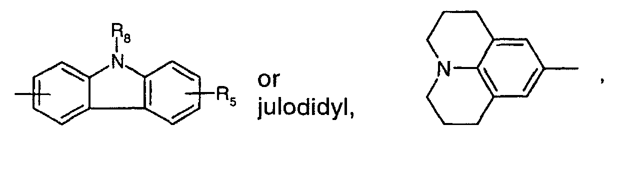 which can be substituted one to four times with C₁-C₄alkyl, C₁-C₄alkoxy, or phenyl or 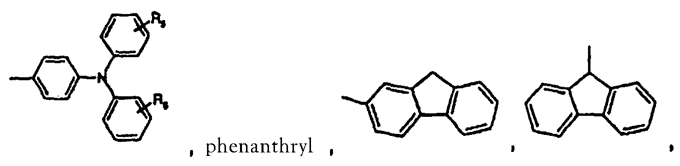 or 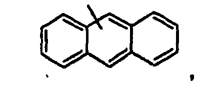 wherein
R₅, R₆ and R₇, independently from each other, stand for hydrogen, cyano, halogen, C₁-C₈alkyl, -NR₈R₉, -OR₁₀, -S(O)ₙR₈, -Se(O)ₙR₈, or phenyl, which can be substituted one to three times with C₁-C₈alkyl or C₁-C₈alkoxy,
wherein R₈ and R₉, independently from each other, stand for hydrogen, phenyl, C₁-C₂₅-alkyl, C₅-C₁₂-cycloalkyl, -CR₃R₄-(CH₂)ₘ-Ph, R₁₀, wherein R₁₀ stands for C₈-C₂₄-aryl, or a saturated or unsaturated heterocyclic radical comprising five to seven ring atoms, wherein the ring consists of carbon atoms and one to three hetero atoms selected from the group consisting of nitrogen, oxygen and sulfur, wherein Ph, the aryl and heterocyclic radical can be substituted one to three times with C₁-C₈alkyl, C₁-C₈alkoxy, or halogen, or R₈ and R₉ stand for -C(O)R₁₁, wherein R₁₁ can be C₁-C₂₅-alkyl, C₅-C₁₂-cycloalkyl, R₁₀, -OR₁₂ or -NR₁₃R₁₄, wherein R₁₂, R₁₃, and R₁₄ stand for C₁-C₂₅-alkyl, C₅-C₁₂-cycloalkyl, C₆-C₂₄-aryl, or
a saturated or unsaturated heterocyclic radical comprising five to seven ring atoms, wherein the ring consists of carbon atoms and one to three hetero atoms selected from the group consisting of nitrogen, oxygen and sulfur, wherein the aryl and heterocyclic radical can be substituted one to three times with C₁-C₈alkyl or C₁-C₈alkoxy, or -NR₈R₉ stands for a five- or sixmembered heterocyclic radical in which R₈ and R₉ together stand for tetramethylene, pentamethylene, -CH₂-CH₂-O-CH₂-CH₂-, or -CH₂-CH₂-NR₅-CH₂-CH₂-, preferably -CH₂-CH₂-O-CH₂-CH₂-, and n stands for 0, 1, 2 or 3, and wherein Z stands for a diradical selected from the group consisting of a single bond, C₂-C₆alkylene, which can be substituted one to three times with C₁-C₄alkyl, C₁-C₄alkoxy, or phenyl, phenylene or naphthylene, with the proviso that R₆ and R₇ do not stand simultaneously for hydrogen.

8. Fluorescent diketopyrrolopyrroles according to claim 7, wherein R₁ = R₂ = C₁-C₈alkyl, Ar₁ = Ar₂ = stilbene, R₇ = -NR₈R₉ in 4-position, R₅ = R₆ = hydrogen, and R₈ = R₉ = C₁-C₈alkyl or phenyl.

9. Fluorescent diketopyrrolopyrroles according to claim 7, wherein R₁ = R₂ = C₁-C₈alkyl, -(CH₂)ₘ-Ph, Ar₁ = Ar₂ = stilbene, R₅ = R₆ = hydrogen, R₇ = -SR₇, -OR₁₀, -N(R₈)₂ or unsubstituted or substituted phenyl in para-position, and R₈ = C₁-C₈alkyl, phenyl or a heterocyclic radical, both unsubstituted or substituted, or C₅-C₁₂-cycloalkyl.

10. Fluorescent diketopyrrolopyrroles according to claim 7, wherein R₁ = R₂ = -CH₂-Ph, wherein phenyl can be substituted with phenyl, naphthyl or C₁-C₄alkyl up to two times, Ar₁ = Ar₂ = 1- or 2-naphthyl, R₅ = R₆ = hydrogen, R₇ = hydrogen or -OMe.

11. Fluorescent diketopyrrolopyrroles according to claim 7:

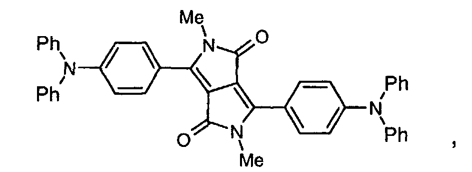

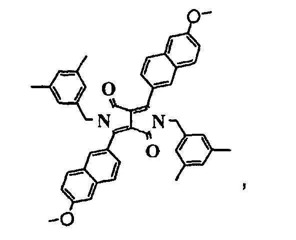

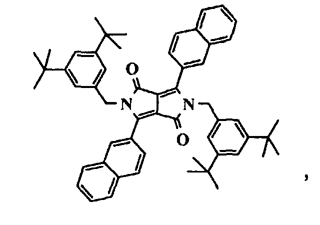

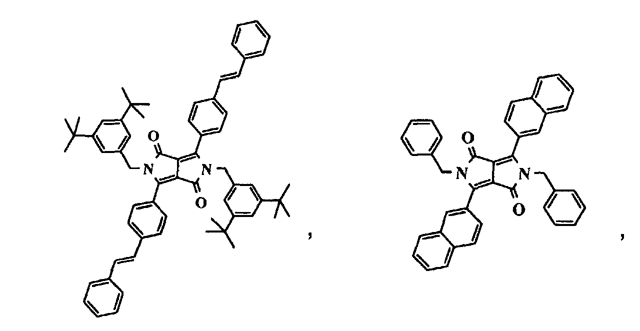

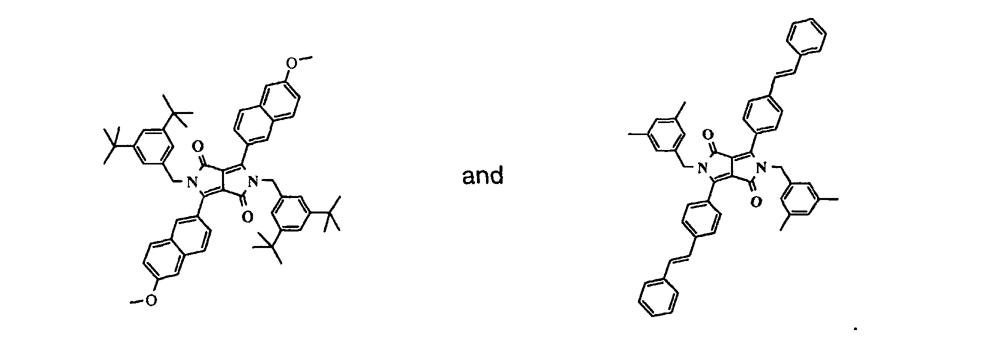

12. Fluorescent diketopyrrolopyrroles of the formula (A2), (A3) or (A4) 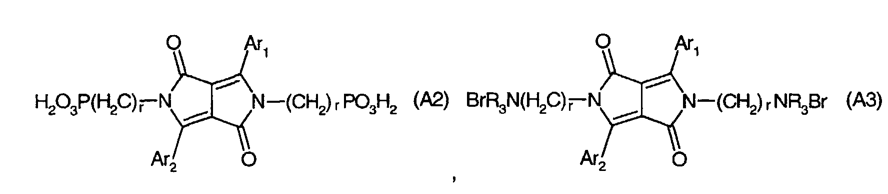 or 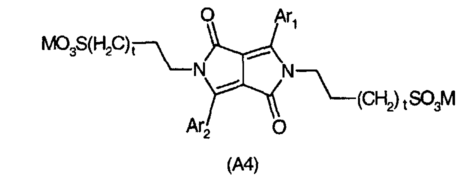 wherein r stands for an integer from 2 to 25; instead of linear alkyl groups, wherein the group -(CH₂)ᵣ- could be replaced by branched alkyl groups or aralkyl groups, such as -(CH₂)ᵣ₁-aryl-(CH₂)ᵣ₂-, r₁ and r₂ being whole numbers in the range of from 0 to 10; wherein M stands for a metal ion such as sodium or potassium, and t is 1 or 2,
Ar₁ and Ar₂, independently from each other, stand for aryl radicals, preferably for 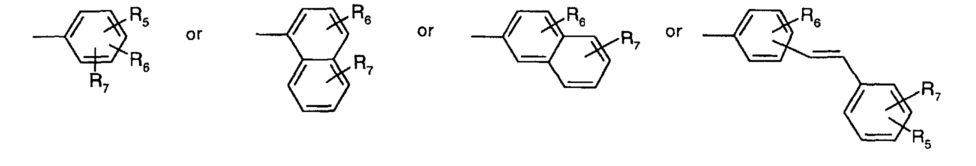 or 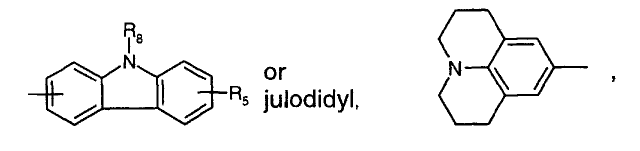 which can be substituted one to four times with C₁-C₄alkyl, C₁-C₄alkoxy, or phenyl or 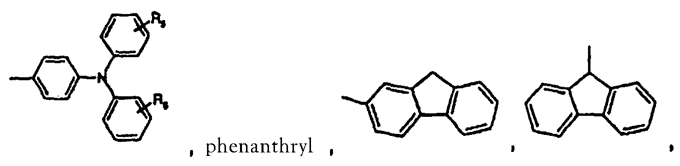 or 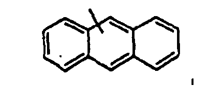 wherein
R₅, R₆ and R₇, independently from each other, stand for hydrogen, cyano, halogen, C₁-C₆alkyl, -NR₈R₉, -OR₁₀, -S(O)ₙR₈, -Se(O)ₙR₈, or phenyl, which can be substituted one to three times with C₁-C₈alkyl or C₁-C₈alkoxy,
wherein R₈ and R₉, independently from each other, stand for hydrogen, phenyl, C₁-C₂₅-alkyl, C₅-C₁₂-cycloalkyl, -CR₃R₄-(CH₂)ₘ-Ph, R₁₀, wherein R₁₀ stands for C₈-C₂₄-aryl, or a saturated or unsaturated heterocyclic radical comprising five to seven ring atoms, wherein the ring consists of carbon atoms and one to three hetero atoms selected from the group consisting of nitrogen, oxygen and sulfur, wherein Ph, the aryl and heterocyclic radical can be substituted one to three times with C₁-C₈alkyl, C₁-C₈alkoxy, or halogen, or
R₈ and R₉ stand for -C(O)R₁₁, wherein R₁₁ can be C₁-C₂₅-alkyl, C₅-C₁₂-cycloalkyl, R₁₀, -R₁₂ or -NR₁₃R₁₄, wherein R₁₂, R₁₃, and R₁₄ stand for C₁-C₂₅-alkyl, C₅-C₁₂-cycloalkyl, C₆-C₂₄-aryl, or a saturated or unsaturated heterocyclic radical comprising five to seven ring atoms, wherein the ring consists of carbon atoms and one to three hetero atoms selected from the group consisting of nitrogen, oxygen and sulfur, wherein the aryl and heterocyclic radical can be substituted one to three times with C₁-C₈alkyl or C₁-C₈alkoxy,
or -NR₈R₉ stands for a five- or sixmembered heterocyclic radical in which R₈ and R₉ together stand for tetramethylene, pentamethylene, -CH₂-CH₂-O-CH₂-CH₂-, or -CH₂-CH₂-NR₅-CH₂-CH₂-, preferably -CH₂-CH₂-O-CH₂-CH₂-, and n stands for 0, 1, 2 or 3.

## Patentansprüche

1. Elektrolumineszierende Vorrichtung, umfassend in dieser Reihenfolge
(a) eine Anode
(b) eine Lochtransportschicht
(c) eine Lichtemissionsschicht
(d) gegebenenfalls eine Elektronentransportschicht und
(e) eine Kathode
und eine Licht emittierende Substanz, wobei die Licht emittierende Substanz ein Diketopyrrolopyrrol ("DPP"), wiedergegeben durch Formel I oder Formel III 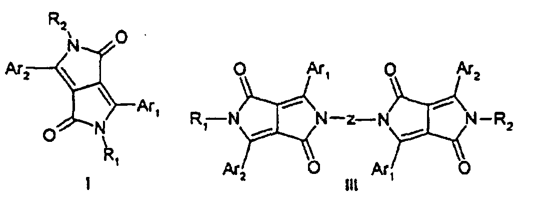 ist, worin R₁ und R₂ unabhängig voneinander stehen für C₁-C₂₅-Alkyl, Allyl, das ein- bis dreifach substituiert sein kann mit C₁-C₃-Alkyl oder Ar₃, oder -CR₃R₄-(CH₂)ₘ-Ar₃, worin R₃ und R₄ unabhängig voneinander für Wasserstoff oder C₁-C₄-Alkyl stehen, oder Phenyl, das mit C₁-C₃-Alkyl ein- bis dreifach substituiert sein kann,
Ar₃ für Phenyl oder 1- oder 2-Naphthyl, welches mit C₁-C₈-Alkyl, C₁-C₈-Alkoxy, Halogen oder Phenyl, welches mit C₁-C₈-Alkyl oder C₁-C₈-Alkoxy ein- bis dreifach substituiert sein kann, ein- bis dreifach substituiert sein kann, steht, und m für 0, 1, 2, 3 oder 4 steht,
Ar₁ und Ar₂ unabhängig voneinander für 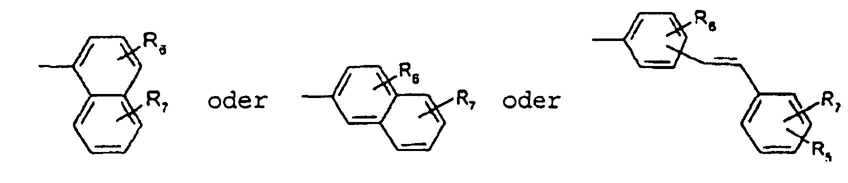 oder 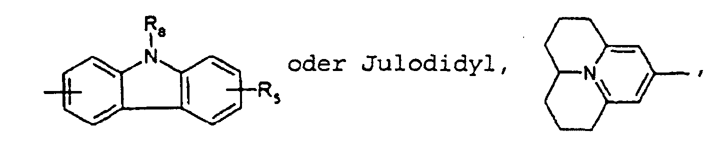 welches ein- bis vierfach substituiert sein kann mit C₁-C₄-Alkyl, C₁-C₄-Alkoxy oder Phenyl, oder 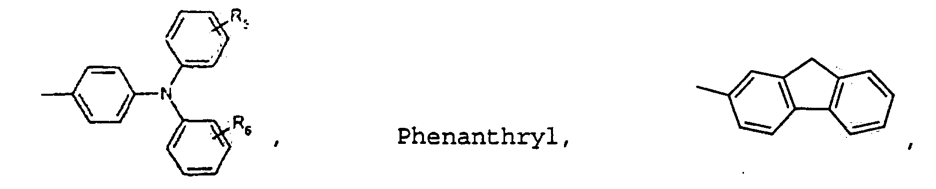 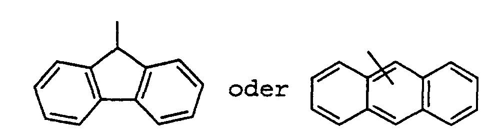 stehen, worin R₅, R₆ und R₇ unabhängig voneinander stehen für Wasserstoff, Cyano, Halogen, C₁-C₆-Alkyl, -NR₈R₉, -OR₁₀, -S(O)ₙR₈, -Se(O)ₙR₈ oder Phenyl, welches mit C₁-C₈-Alkyl oder C₁-C₈-Alkoxy ein- bis dreifach substituiert sein kann,
worin R₈ und R₉ unabhängig voneinander stehen für Wasserstoff, Phenyl, C₁-C₂₅-Alkyl, C₅-C₁₂-Cycloalkyl, -CR₃R₄-(CH₂)ₘ-Ph, R₁₀, worin R₁₀ steht für C₆-C₂₄-Aryl oder einen gesättigten oder ungesättigten heterocyclischen Rest, umfassend fünf bis sieben Ringatome, wobei der Ring besteht aus Kohlenstoffatomen und ein bis drei Heteroatomen, ausgewählt aus der Gruppe, bestehend aus Stickstoff, Sauerstoff und Schwefel, worin Ph, der Aryl- und heterocyclische Rest mit C₁-C₈-Alkyl, C₁-C₈-Alkoxy oder Halogen ein- bis dreifach substituiert sein können oder R₈ und R₉ stehen für -C(O)R₁₁, wobei R₁₁ C₁-C₂₅-Alkyl, C₅-C₁₂-Cycloalkyl, R₁₀, -OR₁₂ oder -NR₁₃R₁₄ sein kann, worin R₁₂, R₁₃ und R₁₄ stehen für C₁-C₂₅-Alkyl, C₅-C₁₂-Cycloalkyl, C₆-C₂₄-Aryl, oder
einen gesättigten oder ungesättigten heterocyclischen Rest, umfassend fünf bis sieben Ringatome, wobei der Ring besteht aus Kohlenstoffatomen und ein bis drei Heteroatomen, ausgewählt aus der Gruppe, bestehend aus Stickstoff, Sauerstoff und Schwefel, wobei der Aryl- und heterocyclische Rest ein- bis dreifach substituiert sein kann mit C₁-C₈-Alkyl oder C₁-C₈-Alkoxy oder -NR₈R₉ steht für einen fünf- oder sechsgliedrigen heterocyclischen Rest, worin R₈ und R₉ zusammen für Tetramethylen, Pentamethylen, -CH₂-CH₂-O-CH₂-CH₂- oder -CH₂-CH₂-NR₅-CH₂-CH₂-, vorzugsweise -CH₂-CH₂-O-CH₂-CH₂-, stehen und n für 0, 1, 2 oder 3 steht, und worin Z für einen Direst, ausgewählt aus der Gruppe, bestehend aus einer Einfachbindung, C₂-C₆-Alkylen, welches mit C₁-C₄-Alkyl, C₁-C₄-Alkoxy oder Phenyl ein- bis dreifach substituiert sein kann, Phenylen oder Naphthylen steht.

2. Verfahren zur Herstellung von Verbindungen I oder III nach Anspruch 1 beim Behandeln in einem ersten Schritt des DPP-Derivats der Formel Va oder Vb 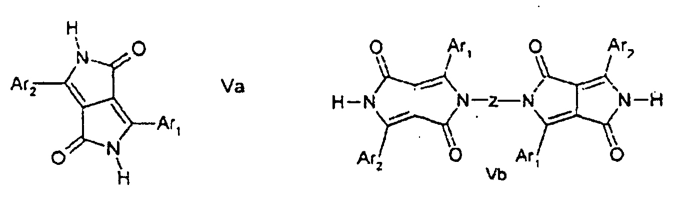 worin Ar₁ und Ar₂ wie in Anspruch 1 definiert sind, mit einer Base, dann in einem zweiten Schritt Behandeln des in dem ersten Schritt erhaltenen Reaktionsgemisches mit einem Alkylierungsmittel, wobei in dem ersten Schritt die Base ein Hydrid, ein Alkalimetallalkoxid oder ein Carbonat darstellt, und das Alkylierungsmittel eine Sulfonat-, Tosylat-, Mesylat-, Carbonat-, Sulfat- oder Halogenverbindung der Formel (R₁)_{1 oder 2}X darstellt, worin X steht für SO₃-, (p-Me-Phenyl)SO₂-, (2,4,6-Trimethyl-phenyl)-SO₂-, -CO₃-, -SO₄- oder Halogen oder ein Gemisch von (R₁)_{1 oder 2}X und (R₂)_{1 oder 2}X.

3. Verfahren zur Herstellung von Verbindungen I nach Anspruch 1, umfassend Behandeln des DPP-Derivats der Formel VIa oder Formel VIb 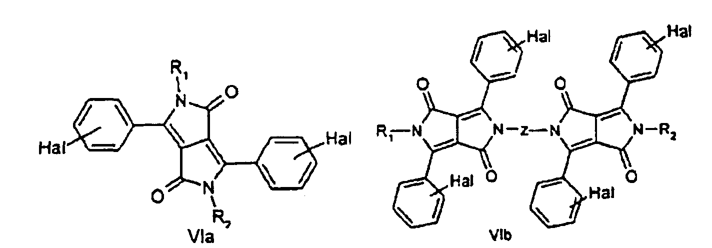 mit einem nucleophilen Mittel, wie einem sekundären Amin, HNR₈R₉, einem Thiol, HSR₈ oder HS(O)ₙR₈, einem Alkohol, HOR₁₀, einem Diselenid, R₈(O)ₙSe-Se(O)ₙR₈, vorzugsweise in einem Molverhältnis von DPP VIa oder VIb:nucleophilem Mittel im Bereich von 1,2:1 bis 0,8:1, oder wenn R₂ die gleiche Bedeutung wie R₁ aufweist, im Bereich von 1:2,5 bis 1:1 in Gegenwart eines. wasserfreien, dipolaren, aprotischen Lösungsmittels und einer wasserfreien Base in einer Menge im Bereich von gewöhnlich 0,1 bis 15 Mol pro Mol des nucleophilen Mittels bei einer Temperatur im Bereich von 100 bis 220°C und unter einem Druck im Bereich von 100 bis 300 kPa und gegebenenfalls Isolieren der erhaltenen Verbindung Va bzw. Vb, worin R₁, R₂, R₈, R₉ und R₁₀ wie in Anspruch 7 definiert sind und Hal für Halogen steht.

4. Verfahren zum Färben von organischen Materialien mit hohem Molekulargewicht durch Einarbeiten der DPP-Verbindungen I oder III nach Anspruch 1 in die Materialien in analoger Weise zu bekannten Verfahren.

5. Zusammensetzung, umfassend:
(a) 0,01 bis 50 Gewichtsprozent, bezogen auf das Gesamtgewicht des gefärbten organischen Materials mit hohem Molekulargewicht, eines fluoreszierenden DPP I oder III nach Anspruch 1, und
(b) 99,99 bis 50 Gewichtsprozent, bezogen auf das Gesamtgewicht des gefärbten organischen Materials mit hohem Molekulargewicht, eines organischen Materials mit hohem Molekulargewicht, und
(c) 0 bis 50 Gewichtsprozent übliche Additive.

6. Zusammensetzung nach Anspruch 5, worin das organische Material mit hohem Molekulargewicht ein Polyamid, ein Polystyrol, vorzugsweise hochschlagfestes Polystyrol, Polymethylmethacrylat oder ein ABS-Copolymer ist.

7. Fluoreszierende, durch Formel I oder Formel III wiedergegebene Diketopyrrolopyrrole 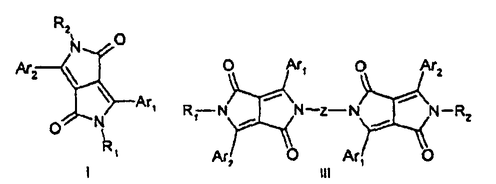 worin R₁ und R₂ unabhängig voneinander stehen für C₁-C₂₅-Alkyl, Allyl, das ein- bis dreifach substituiert sein kann mit C₁-C₃-Alkyl oder Ar₃, oder -CR₃R₄-(CH₂)ₘ-Ar₃, worin R₃ und R₄ unabhängig voneinander für Wasserstoff oder C₁-C₄-Alkyl stehen, oder Phenyl, das mit C₁-C₃-Alkyl ein- bis dreifach substituiert sein kann,
Ar₃ für Phenyl oder 1- oder 2-Naphthyl, welches mit C₁-C₈-Alkyl, C₁-C₈-Alkoxy, Halogen oder Phenyl, welches mit C₁-C₈-Alkyl oder C₁-C₈-Alkoxy ein- bis dreifach substituiert sein kann, ein- bis dreifach substituiert sein kann, steht, und m für 0, 1, 2, 3 oder 4 steht,
Ar₁ und Ar₂ unabhängig voneinander für 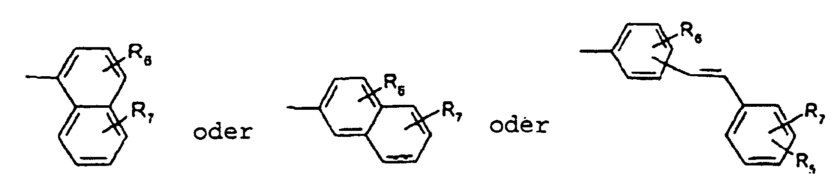 oder 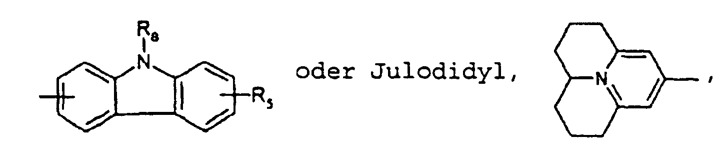 welches ein- bis vierfach substituiert sein kann mit C₁-C₄-Alkyl, C₁-C₄-Alkoxy oder Phenyl, oder 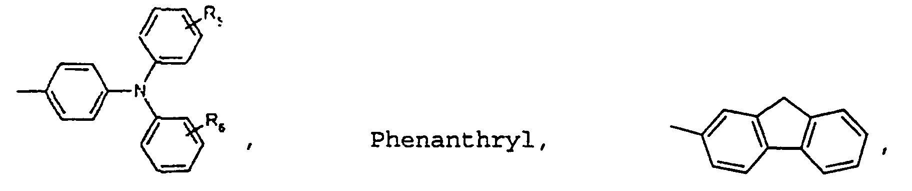 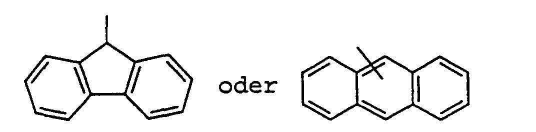 stehen, worin R₅, R₆ und R₇ unabhängig voneinander stehen für Wasserstoff, Cyano, Halogen, C₁-C₆-Alkyl, -NR₈R₉, -OR₁₀, -S(O)ₙR₈, -Se(O)ₙR₈ oder Phenyl, welches mit C₁-C₈-Alkyl oder C₁-C₈-Alkoxy ein- bis dreifach substituiert sein kann,
worin R₈ und R₉ unabhängig voneinander stehen für Wasserstof f , Phenyl, C₁-C₂₅-Alkyl, C₅-C₁₂-Cycloalkyl, -CR₃R₄-(CH₂)ₘ-Ph, R₁₀, worin R₁₀ steht für C₆-C₂₄-Aryl oder einen gesättigten oder ungesättigten heterocyclischen Rest, umfassend fünf bis sieben Ringatome, worin der Ring besteht aus Kohlenstoffatomen und ein bis drei Heteroatomen, ausgewählt aus der Gruppe, bestehend aus Stickstoff, Sauerstoff und Schwefel, worin Ph, der Aryl- und heterocyclische Rest mit C₁-C₈-Alkyl, C₁-C₈-Alkoxy oder Halogen einbis dreifach substituiert sein können oder R₈ und R₉ stehen für -C(O)R₁₁, wobei R₁₁ C₁-C₂₅-Alkyl, C₅-C₁₂-Cycloalkyl, R₁₀, -OR₁₂ oder -NR₁₃R₁₄ sein kann, worin R₁₂, R₁₃ und R₁₄ stehen für C₁-C₂₅-Alkyl, C₅-C₁₂-Cycloalkyl, C₆-C₂₄-Aryl, oder einen gesättigten oder ungesättigten heterocyclischen Rest, umfassend fünf bis sieben Ringatome, worin der Ring besteht aus Kohlenstoffatomen und ein bis drei Heteroatomen, ausgewählt aus der Gruppe, bestehend aus Stickstoff, Sauerstoff und Schwefel, worin der Aryl- und heterocyclische Rest ein- bis dreifach substituiert sein kann mit C₁-C₈-Alkyl oder C₁-C₈-Alkoxy oder -NR₈R₉ steht für einen fünf- oder sechsgliedrigen heterocyclischen Rest, worin R₈ und R₉ zusammen für Tetramethylen, Pentamethylen, -CH₂-CH₂-O-CH₂-CH₂- oder -CH₂-CH₂-NR₅-CH₂-CH₂-, vorzugsweise -CH₂-CH₂-O-CH₂-CH₂-, stehen und n für 0, 1, 2 oder 3 steht, und worin Z für einen Direst, ausgewählt aus der Gruppe, bestehend aus einer Einfachbindung, C₂-C₆-Alkylen, welches mit C₁-C₄-Alkyl, C₁-C₄-Alkoxy oder Phenyl ein- bis dreifach substituiert sein kann, Phenylen oder Naphthylen steht, mit der Maßgabe, dass R₆ und R₇ nicht gleichzeitig für Wasserstoff stehen.

8. Fluoreszierende Diketopyrrolopyrrole nach Anspruch 7, worin R₁ = R₂ = C₁-C₈-Alkyl, Ar₁ = Ar₂ = Stilben, R₇ = -NR₈R₉ in 4-Position, R₅ = R₆ = Wasserstoff und R₈ = R₉ = C₁-C₈-Alkyl oder Phenyl.

9. Fluoreszierende Diketopyrrolopyrrole nach Anspruch 7, worin R₁ = R₂ = C₁-C₈-Alkyl, -(CH₂)ₘ-Ph, Ar₁ = Ar₂ = Stilben, R₅ = R₆ = Wasserstoff, R₇ = -SR₇, -OR₁₀, -N(R₈)₂ oder unsubstituiertes oder substituiertes Phenyl in Paraposition und R₈ = C₁-C₈-Alkyl, Phenyl oder ein heterocyclischer Rest, beide unsubstituiert oder substituiert, oder C₅-C₁₂-Cycloalkyl.

10. Fluoreszierende Diketopyrrolopyrrole nach Anspruch 7, worin R₁ = R₂ = -CH₂-Ph, worin Phenyl mit Phenyl, Naphthyl oder C₁-C₄-Alkyl bis zu zweifach substituiert sein kann, Ar₁ = Ar₂ = 1- oder 2-Naphthyl, R₅ = R₆ = Wasserstoff, R₇ = Wasserstoff oder -OMe.

11. Fluoreszierende Diketopyrrolopyrrole nach Anspruch 7 : 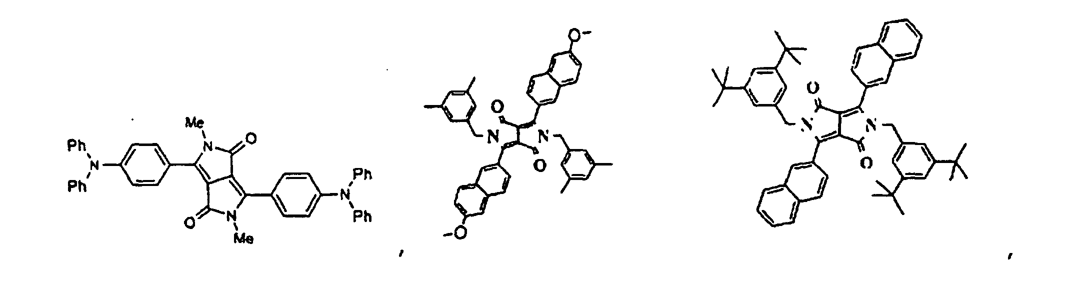 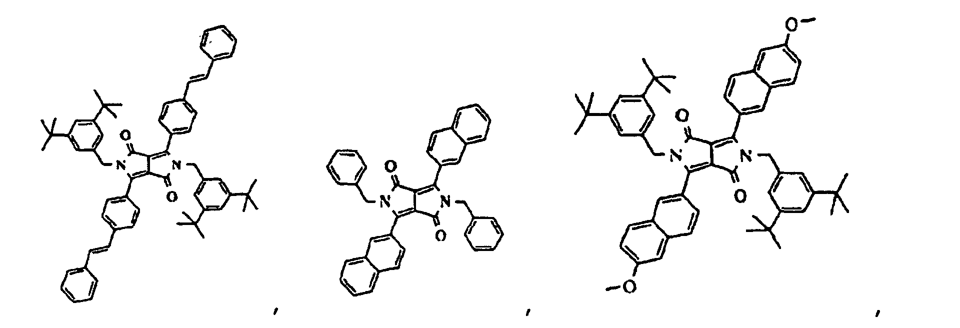 und

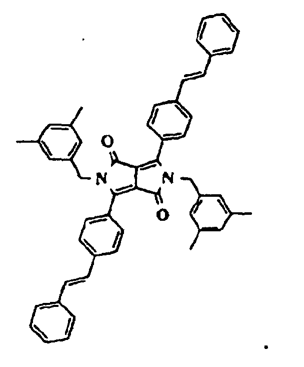

12. Fluoreszierende Diketopyrrolopyrrole der Formel (A2), (A3) oder (A4) 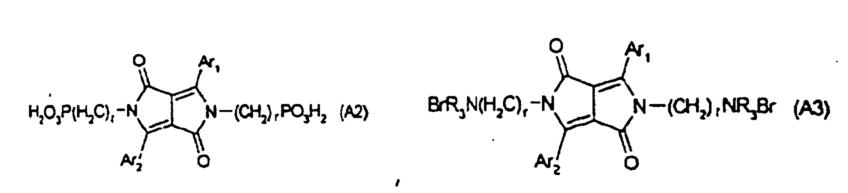 oder 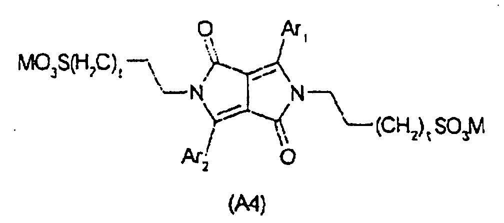 worin r für eine ganze Zahl von 2 bis 25 steht; anstelle von linearen Alkylgruppen, worin die Gruppe -(CH₂)ᵣ- durch verzweigte Alkylgruppen oder Aralkylgruppen wie -(CH₂)ᵣ₁-Aryl-(CH₂)ᵣ₂- ersetzt werden könnten, r₁ und r₂ ganze Zahlen im Bereich von 0 bis 10 sind; worin M für ein Metallion, wie Natrium oder Kalium, steht, und t 1 oder 2 ist, Ar₁ und Ar₂ unabhängig voneinander für Arylreste stehen, vorzugsweise für 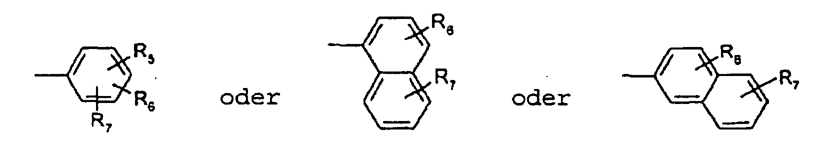 oder 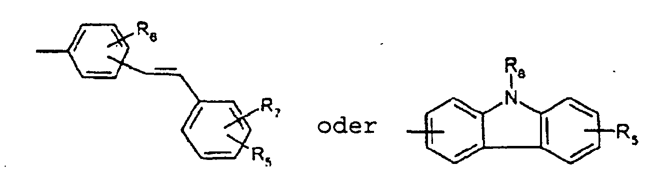 oder Julodidyl, 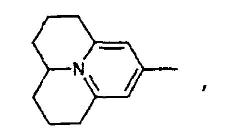 welches ein- bis vierfach substituiert sein kann mit C₁-C₄-Alkyl, C₁-C₄-Alkoxy oder Phenyl, oder 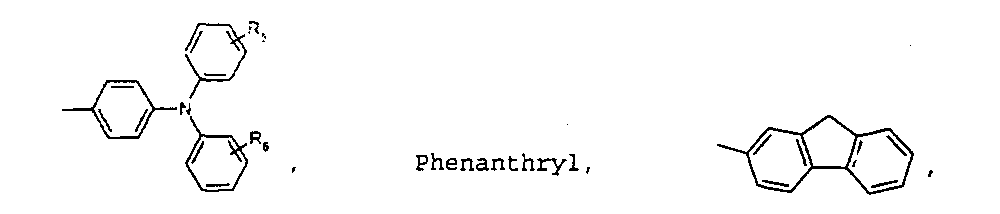 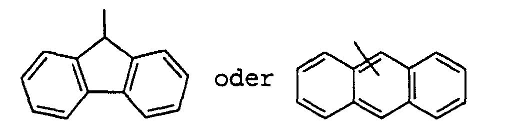 stehen, worin R₅, R₆ und R₇ unabhängig voneinander stehen für Wasserstoff, Cyano, Halogen, C₁-C₆-Alkyl, -NR₈R₉, -OR₁₀, -S(O)ₙR₈, -Se(O)ₙR₈ oder Phenyl, welches mit C₁-C₈-Alkyl oder C₁-C₈-Alkoxy ein- bis dreifach substituiert sein kann,
worin R₈ und R₉ unabhängig voneinander stehen für Wasserstoff, Phenyl, C₁-C₂₅-Alkyl, C₅-C₁₂-Cycloalkyl, -CR₃R₄-(CH₂)ₘ-Ph, R₁₀, worin R₁₀ steht für C₆-C₂₄-Aryl oder einen gesättigten oder ungesättigten heterocyclischen Rest, umfassend fünf bis sieben Ringatome, worin der Ring besteht aus Kohlenstoffatomen und ein bis drei Heteroatomen, ausgewählt aus der Gruppe, bestehend aus Stickstoff, Sauerstoff und Schwefel, worin Ph, der Aryl- und heterocyclische Rest mit C₁-C₈-Alkyl, C₁-C₈-Alkoxy oder Halogen einbis dreifach substituiert sein können oder
R₈ und R₉ stehen für -C(O)R₁₁, wobei R₁₁ C₁-C₂₅-Alkyl, C₅-C₁₂-Cycloalkyl, R₁₀, -R₁₂ oder -NR₁₃R₁₄ sein kann, worin R₁₂, R₁₃ und R₁₄ stehen für C₁-C₂₅-Alkyl, C₅-C₁₂-Cycloalkyl, C₆-C₂₄-Aryl, oder
einen gesättigten oder ungesättigten heterocyclischen Rest, umfassend fünf bis sieben Ringatome, worin der Ring besteht aus Kohlenstoffatomen und ein bis drei Heteroatomen, ausgewählt aus der Gruppe, bestehend aus Stickstoff, Sauerstoff und Schwefel, worin der Aryl- und heterocyclische Rest ein- bis dreifach substituiert sein kann mit C₁-C₈-Alkyl oder C₁-C₈-Alkoxy oder -NR₈R₉ steht für einen fünf- oder sechsgliedrigen heterocyclischen Rest, worin R₈ und R₉ zusammen für Tetramethylen, Pentamethylen, -CH₂-CH₂-O-CH₂-CH₂- oder -CH₂-CH₂-NR₅-CH₂-CH₂-, vorzugsweise -CH₂-CH₂-O-CH₂-CH₂-, stehen und n für 0, 1, 2 oder 3 steht.

## Revendications

1. Dispositif électroluminescent comprenant dans cet ordre
(a) une anode
(b) une couche de transport de lacune
(c) une couche émettrice de lumière
(d) facultativement une couche de transport d'électron et
(e) une cathode
et une substance émettrice de lumière, dans lequel la substance émettrice de lumière est un dicétopyrrolopyrrole ("DPP") représenté par la formule I ou la formule III 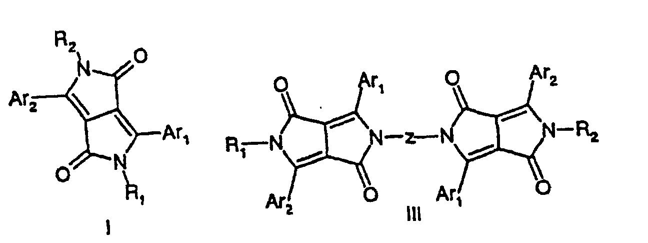 dans lesquelles R₁ et R₂, indépendamment l'un de l'autre, représentent un alkyle en C₁-C₂₅, un allyle qui peut être substitué une à trois fois avec un alkyle en C₁-C₃ ou Ar₃, ou -CR₃R₄-(CH₂)ₘ-Ar₃, dans lequel R₃ et R₄, indépendamment l'un de l'autre, représentent l'hydrogène ou un alkyle en C₁-C₄, ou un phényle qui peut être substitué une à trois fois avec un alkyle en C₁-C₃,
Ar₃ représente le phényle ou le 1- ou 2-naphtyle qui peut être substitué une à trois fois avec un alkyle en C₁-C₆, un alcoxy en C₁-C₆, un halogène ou le phényle, qui peut être substitué avec un alkyle en C₁-C₆ ou un alcoxy en C₁-C₆ une à trois fois, et m représente 0, 1, 2, 3 ou 4,
Ar₁ et Ar₂, indépendamment l'un de l'autre, représentent 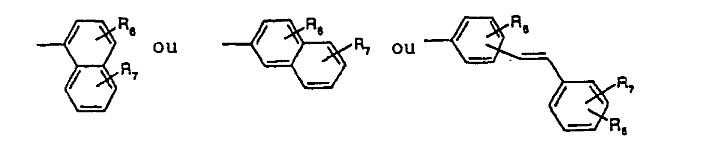 ou 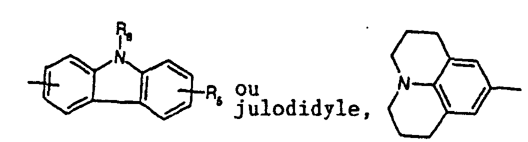 qui peuvent être substitués une à quatre fois avec un alkyle en C₁-C₄, un alcoxy en C₁ à C₄, ou le phényle
ou 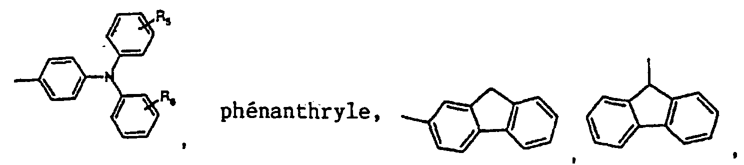 ou 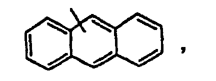 dans lesquels
R₅, R₆ et R₇, indépendamment l'un de l'autre, représentent l'hydrogène, le cyano, un halogène, un alkyle en C₁-C₆, -NR₈R₉, -OR₁₀, -S(O)ₙR₈, -Se(O)ₙR₅, ou le phényle, qui peut être substitué une à trois fois avec un alkyle en C₁-C₆ ou un alcoxy en C₁-C₆,
dans lesquels R₈ et R₉, indépendamment l'un de l'autre, représentent l'hydrogène, le phényle, un alkyle en C₁-C₂₅, un cycloalkyle en C₅-C₁₂, -CR₃R₄-(CH₂)ₘ-Ph, R₁₀ où R₁₀ représente un aryle en C₆-C₂₄, ou un radical hétérocyclique saturé ou insaturé comprenant cinq à sept atomes cycliques, dans lequel le cycle consiste en atomes de carbone et un à trois hétéroatomes choisis dans le groupe consistant en l'azote, l'oxygène et le soufre, dans lequel Ph, l'aryle et le radical hétérocyclique peuvent être substitués une à trois fois avec un alkyle en C₁-C₆, un alcoxy en C₁-C₆, ou un halogène, ou R₈ et R₉ représentent -C(O)R₁₁, dans lequel R₁₁ peut être un alkyle en C₁-C₂₆, un cycloalkyle en C₅-C₁₂, R₁₀, -OR₁₂ ou -NR₁₃R₁₄, dans lesquels R₁₂, R₁₃ et R₁₄ représentent un alkyle en C₁-C₂₆, un cycloalkyle en C₅-C₁₂, un aryle en C₈-C₂₄, ou
un radical hétérocyclique saturé ou insaturé comprenant cinq à sept atomes cycliques, dans lequel le cycle consiste en atomes de carbone et un à trois hétéroatomes choisis dans le groupe consistant en l'azote, l'oxygène et le soufre, dans lequel l'aryle et le radical hétérocyclique peuvent être substitués une à trois fois avec un alkyle en C₁-C₆ ou un alcoxy en C₁-C₆, ou -NR₈R₉ représente un radical hétérocyclique à cinq ou six chaînons dans lequel R₈ et R₉ représentent conjointement le tétraméthylène, le pentaméthylène, -CH₂-CH₂-O-CH₂-CH₂-, ou -CH₂-CH₂-NR₅-CH₂-CH₂-, de préférence -CH₂-CH₂-O-CH₂-CH₂-, et n représente 0, 1, 2 ou 3, et dans lesquelles Z représente un diradical choisi dans le groupe consistant en une simple liaison, un alkylène en C₂-C₆, qui peut être substitué une à trois fois avec un alkyle en C₁-C₄, un alcoxy en C₁-C₄, ou le phényle, le phénylène ou le naphtylène.

2. Procédé pour la préparation de composés I ou III selon la revendication 1 en traitant dans une première étape le dérivé de DPP de formule Va ou de formule Vb 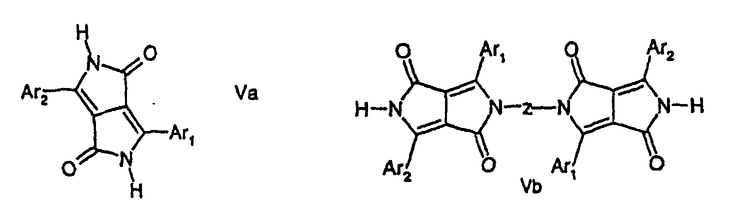 dans lesquelles Ar₁ et Ar₂ sont comme définis dans la revendication 1, avec une base, puis, dans une deuxième étape, en traitant le mélange de réaction obtenu dans la première étape avec un agent d'alkylation, où, dans la première étape, la base est un hydrure, un alcoxyde ou un carbonate de métal alcalin, et l'agent d'alkylation est un sulfonate, un tosylate, un mésylate, un carbonate, un sulfate, ou un composé halogéné de formule (R₁)_{1 ou 2}X, dans laquelle X représente SO₃⁻, (p-Me-phényl)SO₂⁻, (2,4,6-triméthyl-phényl)-SO₂⁻, -CO₃⁻, -SO₄⁻, ou un halogène, ou un mélange de (R₁)₁ _{ou 2}X et de (R₂)_{1 ou 2}X.

3. Procédé pour la préparation de composés I selon la revendication 1 par traitement du dérivé de formule VIa ou de formule VIb 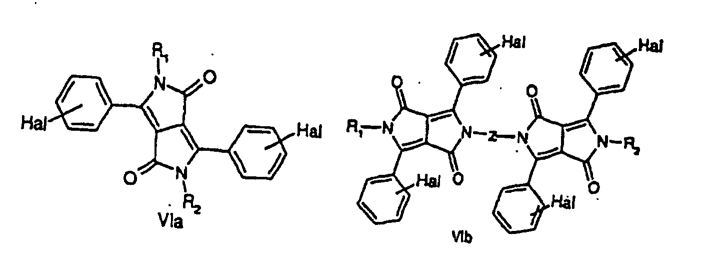 avec un agent nucléophile tel qu'une amine secondaire, HNR₈R₉, un thiol, HSR₈, ou HS(O)ₙR₉, un alcool, HOR₁₀, un disélénure, R₆(O)ₙSe-Se(O)ₐR₈, de préférence en un rapport molaire de DPP VIa ou VIb:agent nucléophile dans l'intervalle de 1,2:1 à 0,8:1, ou, si R₂ a la même définition que R₁ dans l'intervalle de 1:2,5 à 1:1, en présence d'un solvant aprotique dipolaire anhydre, et d'une base anhydre en une quantité dans l'intervalle de généralement 0,1 à 15 moles par mole de l'agent nucléophile, à une température dans l'intervalle de 100 à 220°C et sous une pression dans l'intervalle de 100 à 300 kPa, et facultativement par isolement du composé Va, respectivement Vb obtenu où R₁, R₂, R₈, R₉ et R₁₀ sont comme définis dans la revendication 7 et Hal représente un halogène.

4. Procédé de coloration de matériaux organiques de poids moléculaire élevé par incorporation des composés DPP I ou III selon la revendication 1 dans lesdits matériaux d'une manière analogue à des procédés connus.

5. Composition comprenant
(a) 0,01 à 50 % en poids, sur la base du poids total du matériau organique de poids moléculaire élevé coloré, d'un DPP fluorescent I ou III selon la revendication 1, et
(b) 99,99 à 50 % en poids, sur la base du poids total du matériau organique de poids moléculaire élevé coloré, d'un matériau organique de poids moléculaire élevé, et
(c) 0 à 50 % en poids d'additifs usuels.

6. Composition selon la revendication 5, dans laquelle le matériau organique de poids moléculaire élevé est un polyamide, un polystyrène, de préférence un polystyrène choc, un polyméthylméthacrylate ou un copolymère ABS.

7. Dicétopyrrolopyrroles fluorescents représentés par la formule I ou la formule III 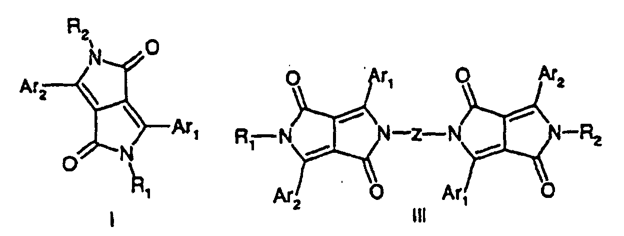 dans lesquelles R₁ et R₂, indépendamment l'un de l'autre, représentent un alkyle en C₁-C₂₅, un allyle qui peut être substitué une à trois fois avec un alkyle en C₁-C₃ ou Ar₃, ou -CR₃R₄-(CH₂)ₘ-Ar₃, dans lequel R₃ et R₄, indépendamment l'un de l'autre, représentent l'hydrogène ou un alkyle en C₁-C₄, ou un phényle qui peut être substitué une à trois fois avec un alkyle en C₁-C₃,
Ar₃ représente le phényle ou le 1- ou 2-naphtyle qui peut être substitué une à trois fois avec un alkyle en C₁-C₆, un alcoxy en C₁-C₆, un halogène ou le phényle, qui peut être substitué avec un alkyle en C₁-C₆ ou un alcoxy en C₁-C₆ une à trois fois, et m représente 0, 1, 2, 3 ou 4,
Ar₁ et Ar₂, indépendamment l'un de l'autre, représentent 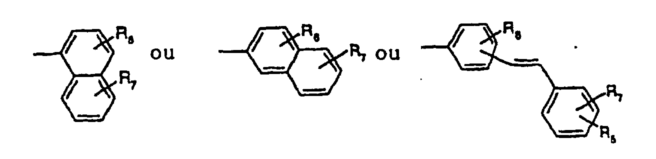 ou 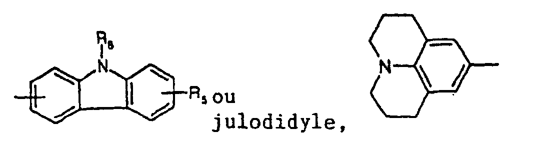 qui peuvent être substitués une à quatre fois avec un alkyle en C₁-C₄, un alcoxy en C₁ à C₄, ou le phényle
ou 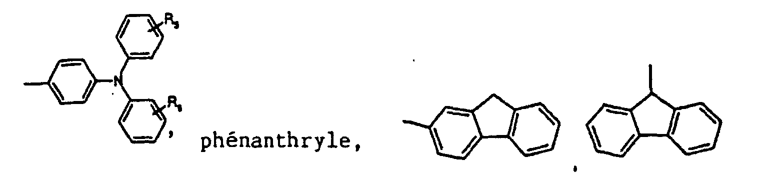 ou 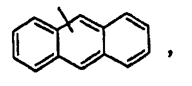 dans lesquels
R₅, R₆ et R₇, indépendamment l'un de l'autre, représentent l'hydrogène, le cyano, un halogène, un alkyle en C₁-C₆, -NR₈R₉, -OR₁₀, -S(O)ₙR₅, -Se(O)ₙR₈, ou le phényle, qui peut être substitué une à trois fois avec un alkyle en C₁-C₆ ou un alcoxy en C₁-C₆,
dans lesquels R₈ et R₉, indépendamment l'un de l'autre, représentent l'hydrogène, le phényle, un alkyle en C₁-C₂₅, un cycloalkyle en C₅-C₁₂, -CR₃R₄-(CH₂)ₘ-Ph, R₁₀ où R₁₀ représente un aryle en C₆-C₂₄, ou un radical hétérocyclique saturé ou insaturé comprenant cinq à sept atomes cycliques, dans lequel le cycle consiste en atomes de carbone et un à trois hétéroatomes choisis dans le groupe consistant en l'azote, l'oxygène et le soufre, dans lequel Ph, l'aryle et le radical hétérocyclique peuvent être substitués une à trois fois avec un alkyle en C₁-C₆, un alcoxy en C₁-C₆, ou un halogène, ou
R₈ et R₉ représentent -C(O)R₁₁, dans lequel R₁₁ peut être un alkyle en C₁-C₂₆, un cycloalkyle en C₅-C₁₂, R₁₀, -OR₁₂ ou -NR₁₃R₁₄, dans lesquels R₁₂, R₁₃ et R₁₄ représentent un alkyle en C₁-C₂₆, un cycloalkyle en C₅-C₁₂, un aryle en C₈-C₂₄, ou
un radical hétérocyclique saturé ou insaturé comprenant cinq à sept atomes cycliques, dans lequel le cycle consiste en atomes de carbone et un à trois hétéroatomes choisis dans le groupe consistant en l'azote, l'oxygène et le soufre, dans lequel l'aryle et le radical hétérocyclique peuvent être substitués une à trois fois avec un alkyle en C₁-C₆ ou un alcoxy en C₁-C₆, ou -NR₈R₉ représente un radical hétérocyclique à cinq ou six chaînons dans lequel R₈ et R₉ représentent conjointement le tétraméthylène, le pentaméthylène, -CH₂-CH₂-O-CH₂-CH₂-, ou -CH₂-CH₂-NR₅-CH₂-CH₂-, de préférence -CH₂-CH₂-O-CH₂-CH₂-, et n représente 0, 1, 2 ou 3, et dans lesquelles Z représente un diradical choisi dans le groupe consistant en une simple liaison, un alkylène en C₂-C₆, qui peut être substitué une à trois fois avec un alkyle en C₁-C₄, un alcoxy en C₁-C₄, ou le phényle, le phénylène ou le naphtylène, à condition que R₈ et R₇ ne représentent pas simultanément l'hydrogène.

8. Dicétopyrrolopyrroles fluorescents selon la revendication 7, dans lesquels R₁ = R₂ = alkyle en C₁-C₆, Ar₁ = Ar₂ = stilbène, R₇ = -NR₈R₉ en position 4, R₅ = R₆ = hydrogène, et R₈ = R₉ = alkyle en C₁-C₆ ou le phényle.

9. Dicétopyrrolopyrroles fluorescents selon la revendication 7, dans lesquels R₁ = R₂ = alkyle en C₁-C₆, -(CH₂)ₘ-Ph, Ar₁ = Ar₂ =stilbène, R₆ = R₈ = hydrogène, R₇ = -SR₇, -OR₁₀, -N(R₈)₂ ou un phényle substitué ou non substitué en position para, et R₈ = alkyle en C₁-C₆, le phényle ou un radical hétérocyclique, tous deux substitués ou non substitués, ou un cycloalkyle en C₅-C₁₂.

10. Dicétopyrrolopyrroles fluorescents selon la revendication 7, dans lesquels R₁ = R₂ = -CH₂-Ph, où le phényle peut être substitué avec le phényle, le naphtyle ou un alkyle en C₁-C₄ jusqu'à deux fois, Ar₁ = Ar₂ = 1- ou 2-naphtyle, R₆ = R₈ = hydrogène, R₇ = hydrogène ou -OMe.

11. Dicétopyrrolopyrroles fluorescents selon la revendication 7 : 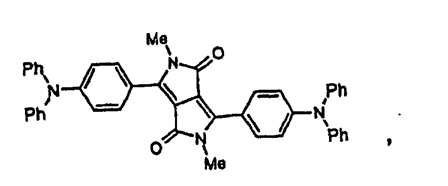 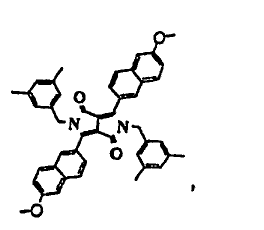 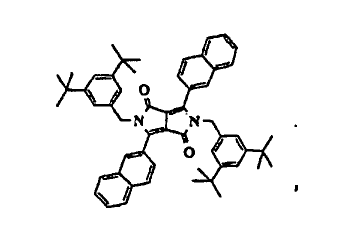 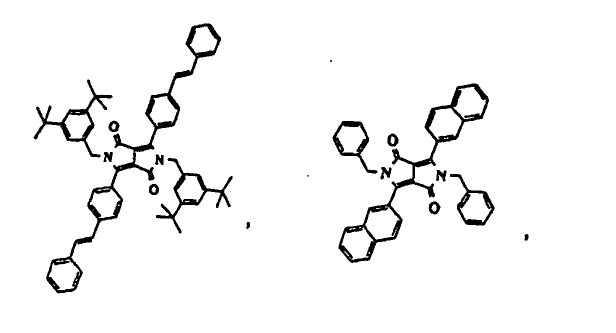 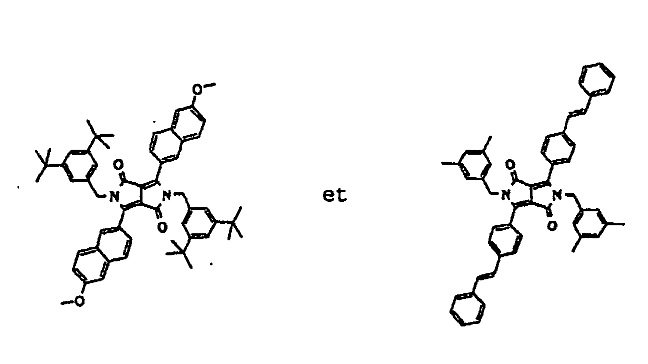 et

12. Dicétopyrrolopyrroles fluorescents de formule (A2), (A3) ou (A4) 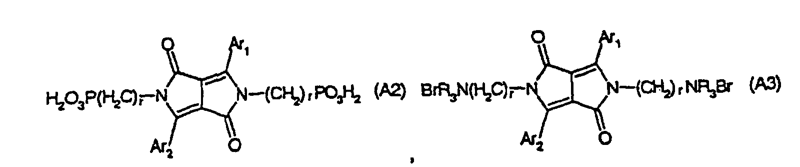 ou 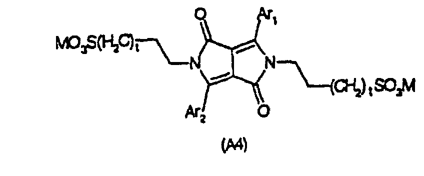 dans lesquelles r représente un entier de 2 à 25 ; au lieu de groupes alkyles linéaires, dans lesquels le groupe -(CH₂)ᵣ- peut être remplacé par des groupes alkyles
ou aralkyles ramifiés, tels que (CH₂)ᵣ₁-aryl-(CH₂)r₂, r₁ et r₂ étant des nombres entiers dans l'intervalle de 2 à 10 ; où M représente un ion de métal tel que le sodium ou le potassium, et t est 1 ou 2,
Ar₁ et Ar₂, indépendamment l'un de l'autre, représentent 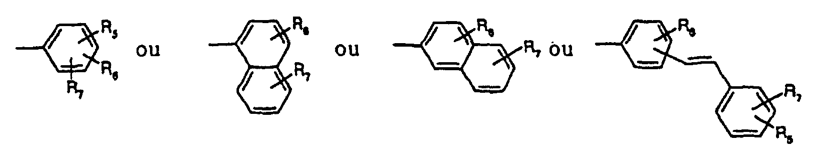 ou 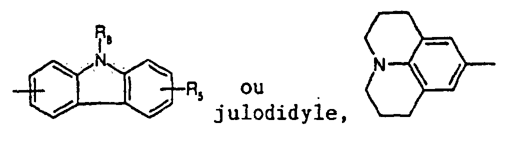 qui peuvent être substitués une à quatre fois avec un alkyle en C₁-C₄, un alcoxy en C₁ à C₄, ou le phényle
ou 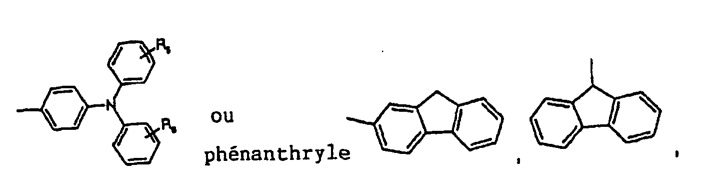 ou 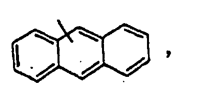 dans lesquels
R₅, R₆ et R₇, indépendamment l'un de l'autre, représentent l'hydrogène, le cyano, un halogène, un alkyle en C₁-C₆, -NR₈R₉, -OR₁₀, -S(O)ₙR₅, -Se(O)ₙR₈, ou le phényle, qui peut être substitué une à trois fois avec un alkyle en C₁-C₆ ou un alcoxy en C₁-C₆,
dans lesquels R₈ et R₉, indépendamment l'un de l'autre, représentent l'hydrogène, le phényle, un alkyle en C₁-C₂₅, un cycloalkyle en C₅-C₁₂, -CR₃R₄-(CH₂)ₘ-Ph, R₁₀ où R₁₀ représente un aryle en C₆-C₂₄, ou un radical hétérocyclique saturé ou insaturé comprenant cinq à sept atomes cycliques, dans lequel le cycle consiste en atomes de carbone et un à trois hétéroatomes choisis dans le groupe consistant en l'azote, l'oxygène et le soufre, dans lequel Ph, l'aryle et le radical hétérocyclique peuvent être substitués une à trois fois avec un alkyle en C₁-C₆, un alcoxy en C₁-C₆, ou un halogène, ou
R₈ et R₉ représentent -C(O)R₁₁, dans lequel R₁₁ peut être un alkyle en C₁-C₂₆, un cycloalkyle en C₅-C₁₂, R₁₀, -R₁₂ ou -NR₁₃R₁₄, dans lesquels R₁₂, R₁₃ et R₁₄ représentent un alkyle en C₁-C₂₆, un cycloalkyle en C₅-C₁₂, un aryle en C₈-C₂₄, ou un radical hétérocyclique saturé ou insaturé comprenant cinq à sept atomes cycliques, dans lequel le cycle consiste en atomes de carbone et un à trois hétéroatomes choisis dans le groupe consistant en l'azote, l'oxygène et le soufre, dans lequel l'aryle et le radical hétérocyclique peuvent être substitués une à trois fois avec un alkyle en C₁-C₆ ou un alcoxy en C₁-C₆,
ou -NR₈R₉ représente un radical hétérocyclique à cinq ou six chaînons dans lequel R₈ et R₉ représentent conjointement le tétraméthylène, le pentaméthylène, -CH₂-CH₂-O-CH₂-CH₂-, ou -CH₂-CH₂-NR₅-CH₂-CH₂-, de préférence -CH₂-CH₂-O-CH₂-CH₂-, et n représente 0, 1, 2 ou 3.
